# EUROPÄISCHE PATENTSCHRIFT

(11) **EP 1 246 911 B1**
(45) Veröffentlichungstag und Bekanntmachung des Hinweises auf die Patenterteilung: **12.09.2007**
(21) Anmeldenummer: 99963285.4
(22) Anmeldetag: 04.01.2000
(51) Int. Cl.: C12N 15/10, C12Q 1/68, C12N 15/82

(54) **VERFAHREN ZUR ERKENNUNG UND CHARAKTERISIERUNG VON WIRKSTOFFEN GEGEN PFLANZEN-PATHOGENE**
METHOD FOR DETECTING AND CHARACTERISING ACTIVE AGENTS AGAINST PLANT PATHOGENS
PROCEDE POUR RECONNAITRE ET CARACTERISER DES PRINCIPES ACTIFS CONTRE DES PATHOGENES DE PLANTES

(43) Veröffentlichungstag der Anmeldung: 09.10.2002
(73) Patentinhaber: Felsenstein, Friedrich, 85395 Attenkirchen (DE); Thümmler, Fritz, 85414 Kirchdorf (DE)
(72) Erfinder: Felsenstein, Friedrich, 85395 Attenkirchen (DE); Thümmler, Fritz, 85414 Kirchdorf (DE)
(74) Vertreter: Munk, Ludwig
(86) Internationale Anmeldenummer: PCT/DE1999/004093
(87) Internationale Veröffentlichungsnummer: WO 2001/049843

(56) Entgegenhaltungen:
- WO-A-00/01830
- WO-A-91/05475
- WO-A-93/07279
- DE-A- 19 860 313
- PIETERSE CORNE M J ET AL: "Increased expression of the calmodulin gene of the late blight fungus Phytophthora infestans during pathogenesis on potato." MOLECULAR PLANT-MICROBE INTERACTIONS, Bd. 6, Nr. 2, 1993, Seiten 164-172, XP000950045 ISSN: 0894-0282
- FIDANTSEF ANA L ET AL: "Characterization of potato tuber lipoxygenase cDNAs and lipoxygenase expression in potato tubers and leaves." PHYSIOLOGIA PLANTARUM, Bd. 102, Nr. 2, Februar 1998 (1998-02), Seiten 257-271, XP000951494 ISSN: 0031-9317
- DIATCHENKO L ET AL: "SUPPRESSION SUBTRACTIVE HYBRIDIZATION: A METHOD FOR GENERATING DIFFERENTIALLY REGULATED OR TISSUE-SPECIFIC CDNA PROBES AND LIBRARIES" PROCEEDINGS OF THE NATIONAL ACADEMY OF SCIENCES OF USA,US,NATIONAL ACADEMY OF SCIENCE. WASHINGTON, Bd. 93, 1. Juni 1996 (1996-06-01), Seiten 6025-6030, XP002911922 ISSN: 0027-8424 in der Anmeldung erwähnt

## Beschreibung

Die Erfindung betrifft ein Verfahren zur Erkennung und/oder zur Charakterisierung der anti-pflanzenpathogenen Wirksamkeit von Agenzien, insbesondere ein Screening-Verfahren für derartige Agenzien.

Es handelt sich dabei um ein *in vivo* -Verfahren, bei dem die Interaktion zwischen Pflanze und Pathogen auf Genebene analysiert wird. Analysiert wird die differentielle Expression von Genen, deren Expression durch Pathogeninfektion verändert wird. Der Verlauf und die Stärke der Interaktion zwischen Wirt und Pathogen dient als Parameter zur Abschätzung der antipflanzenpathogenen Wirksamkeit einer Substanz. Die Betrachtung ist dabei im Rahmen der Erfindung nicht auf das Pathogen fixiert, sondern berücksichtigt in hohem Ausmaß den Wirtsorganismus und seine entsprechenden Reaktionen.

Der Wirtsorganismus und seine infektionsabhängige differentielle Genexpression sind ein wichtiger Informationsbestandteil für die Erkennung und Charakterisierung der antipflanzenpathogenen Wirksamkeit eines Agens.

Die bisher angewandten Verfahren zur Erkennung pathogenwirksamer Substanzen, d.h. von gegen ein Pathogen wirkenden Agenzien, zielen vornehmlich darauf ab, anhand der Reaktion des Erregers die Wirksamkeit der Substanzen zu erkennen und zu quantifizieren. Im Mittelpunkt der bisherigen Betrachtungsweise gemäß dem Stand der Technik steht folglich das Pathogen, insbesondere sein Wachstum, seine Entwicklung, Ausbreitung und erkennbare Symptomausprägung.

Bei fakultativen Pathogenen werden die Untersuchungen im sog. Massen-Screening zumeist an den Pathogenen in geeigneten Nährmedien vorgenommen, die mit der zu untersuchenden Substanz versetzt sind. Hierzu dient vielfach das Mikrotiter-Verfahren. Eine Beschreibung findet sich in PIJLS et al., (1994) 'A rapid test to evaluate in vitro sensitivity of Septoria tritici to flutriafol using a microtitre plate reader' (Plant Pathology 43, 726-732). Hierbei ergeben sich jedoch oftmals grundlegende Probleme: Zum einen können aufgrund der methodischen Schwankungsbreite die Substanzen nicht immer als wirksam identifiziert werden, zum anderen sind die Befunde über etwaige potentielle Wirkstoffe nicht automatisch auf die Pflanze übertragbar, da hier noch eine ganze Reihe weiterer Faktoren wie beispielsweise die etwaige Umwandlung einer Substanz im Wirtsorganismus (Metabolismus) die Wirksamkeit positiv oder negativ beeinflussen können. Folgeuntersuchungen an der Pflanze im Gewächshaus oder im Feld können daher zu Ergebnissen führen, die den Ergebnissen der *in vitro*-Versuche widersprechen. Fehleinschätzungen und -planungen sowie größere ökonomische Einbußen sind damit die zwangsläufige Folge.

WO 93/07279 offenbart ein Verfahren zum Identifizieren induzierbarer Abwehrgene und -promotoren von Pflanzen. Zusätzlich beschreibt diese Druckschrift ein Verfahren zum Identifizieren bestimmter Agrochemikalien, die derartige Pflanzenabwehrgene induzieren, insbesondere derartige Agrochemikalien, die regulatorische Elemente dafür aus Pflanzen unmittelbar oder kurz nach einer entsprechenden Exposition induzieren.

Molecular Plant Microbe Interactions [Bd. 6, S. 164 - 172 (1993)] offenbart die etwa 5-fach erhöhte Expression des P. infestans Calmodulin-Gens während der Pathogenese der Kartoffelfäule auf den Blättern der Kartoffel.

Zudem stehen für das Folgescreening an der Pflanze in der Regel nur sehr geringe Substanzmengen zur Verfügung, wobei gleichzeitig die Anzahl der zu prüfenden Substanzen möglichst hoch sein soll. Beide Gesichtspunkte sind mit dem herkömmlichen Gewächshausversuchswesen nur schwer in den Griff zu bekommen und limitieren wie ein Flaschenhals den Substanzdurchsatz. Ein Verfahren, das eine Miniaturisierung und Beschleunigung des Prüfsystems erlaubt, böte hier erhebliche Vorteile.

Bei obligaten Erregern erfolgt auch das Massenscreening an lebendem Pflanzenmaterial. Dabei werden als Parameter die Entwicklung, das Wachstum, die Symptomausprägung oder die Reproduktion des Krankheitserregers herangezogen. Der von Pathogen zu Pathogen unterschiedlich hohe Zeitbedarf bis zur Symptomausprägung ist als ein Nachteil dieser Methode anzuführen. Des weiteren unterliegt die Symptomausprägung bei etlichen Krankheitserregern (z.B. Rostkrankheiten) oftmals einem breiteren Schwankungsbereich, was eine korrekte Beurteilung der Substanz erheblich erschwert. Eine Miniaturisierung der bisherigen Systeme ist zudem nur schwer zu realisieren, so daß der Umgang mit den nur in kleinen Mengen zur Verfügung stehenden Probesubstanzen ebenfalls ein limitierendes Problem darstellt.

Weitere Probleme ergeben sich aus der Art der Auswertung der Biotests; diese erfolgt zumeist von Auge durch hierfür extra geschulte Personen. Dabei müssen eine Vielzahl von Proben in kurzer Zeit bonitiert werden und die untersuchende Person muß schnell und spontan den Infektionsgrad der Proben visuell erkennen - ein Nacharbeiten der subjektiven Ergebnisse ist zu einem späteren Zeitpunkt in der Regel nicht mehr möglich.

Muß zur Beurteilung der Wirksamkeit einer Substanz bis zur Symtomausprägung gewartet werden, so birgt das noch einen zusätzlichen, ganz erheblichen Nachteil in sich, nämlich die Kreuzkontaminationsgefahr benachbarter Testansätze. Bisher konnte einer derartigen Kontaminationsgefahr nur durch erhöhten technischen Aufwand entgegengewirkt werden, wobei eine klare räumliche Trennung zumeist mit einem sehr hohen Platzbedarf verbunden ist. Auch mit hohem Aufwand ist jedoch mit der bisherigen Methode die Gefahr der Kreuzkontamination nicht ganz zu vermeiden und diese stellt somit einen nicht zu unterschätzenden limitierenden Faktor dar.

Unter Berücksichtigung des Standes der Technik und der vorstehend genannten, dabei auftretenden Probleme liegt der Erfindung die Aufgabe zugrunde, ein schnelles, kostengünstiges und zuverlässiges Verfahren zur Untersuchung der anti-pflanzenpathogenen Wirksamkeit von Agenzien bereitzustellen, das die vorstehend erwähnten Nachteile der gängigen Untersuchungsverfahren vermeidet. Es soll insbesondere auch für Screening-Verfahren verwendbar sein.

Diese Aufgabe wird durch ein Verfahren, insbesondere Screening-Verfahren, zur Erkennung und/oder Charakterisierung der anti-pflanzenpathogenen Wirksamkeit von Agenzien gelöst, wobei die Erkennung bzw. die Charakterisierung der Wirksamkeit anhand der Veränderung der Expressionsstärke der Gene auf molekularer Ebene bei Infektion von Pflanzen mit Pathogenen erfolgt.

Mit dem erfindungsgemäßen Verfahren können die Interaktionen von Pflanze und Pathogen auf Ebene der Genexpression von Beginn an qualitativ und quantitativ bestimmt werden. Der Vorteil des Verfahrens liegt darin, daß die infektionsabhängig veränderte Genexpression (differentielle Genexpression) sowohl seitens des Wirts (Pflanze) als auch seitens des Erregers sichtbar gemacht werden kann. Das Verfahren kann zur Aufdeckung und näheren Charakterisierung wirksamer Substanzen gegen Pathogene genutzt werden. Die gewonnenen - molekularen - Daten sind eine hervorragende Informationsgrundlage zur raschen und sicheren Abschätzung der Wirksamkeit der untersuchten Substanzen. Mit Hilfe der Ergebnisse lassen sich die Nachteile der bisher genutzten Verfahren (Fehleinschätzungen u.a., s.o.) vermeiden. Man befindet sich auf der untersten Reaktionsebene (Erkennungs- und Genaktivierungsebene) der Infektion (Wirt-Parasit-Interaktion). Man ist damit beispielsweise nicht mehr auf eine sichtbare (makroskopische bzw. mikroskopische) Symptomausprägung durch den Krankheitserreger mit all den damit verbundenen Schwierigkeiten und Unzulänglichkeiten angewiesen. Mit dem erfindungsgemäßen Verfahren in seinen verschiedenen Aspekten ergeben sich klar quantifizierbare Unterschiede in der Intensität der Ausprägung der molekulargenetischen Parameter und es läßt sich explizit die Wirksamkeit einer Substanz bestimmen.

Des weiteren kann das erfindungsgemäße Verfahren aufgrund seines differentiellen Ansatzes zur Klärung von Wirkmechanismen der antipathogenen Substanz am Pathogen beitragen, da die Wirt-Pathogen-Interaktion sowohl in ihrer Qualität, Quantität als auch hinsichtlich ihrer Kinetik darstellbar wird. So ließe sich anhand der differentiellen Genexpression beispielsweise aufzeigen, in welchem Infektionsstadium eine antipathogene Substanz auf das Pathogen einwirkt.

Außerdem hat das erfindungsgemäße Verfahren den Vorteil, daß die objektiven *in vivo* Daten direkt von einem Computer gesammelt werden und auch zu einem viel späteren Zeitpunkt beliebig oft ausgewertet und überarbeitet werden können.

Aufgrund der Möglichkeit zur Identifizierung und Charakterisierung infektionsspezifischer Gene auf der Pathogenseite ergeben sich aus dem erfindungsgemäßen Verfahren auch Perspektiven zur Aufklärung von Resistenzmechanismen bei Pathogenen gegenüber Wirkstoffen. Daneben ist die Bestimmung von neuen Targets für das Fungizidscreening denkbar.

Gemäß einem ersten Aspekt des erfindungsgemäßen Verfahrens erfolgt die Erkennung bzw. die Charakterisierung der Wirksamkeit anhand der Genexpression auf der Ebene der RNA.

Eine erste bevorzugte Ausführungsform dieses Verfahrens gemäß dem ersten Aspekt umfaßt die Schritte:
a) Anlegen einer Genbank mit Gensequenzen, deren Expression sich bei Infektion von Pflanzen mit Pathogenen ändert,
b) Behandlung von pflanzlichen Strukturen mit dem auf anti-pflanzenpathogene Wirksamkeit zu prüfenden Agens sowie Behandlung mit dem Pathogen,
c) Isolierung der RNA des Behandlungsansatzes von Schritt b), bevorzugterweise Isolierung der mRNA,
d) Herstellung einer Hybridisierungssonde zu der RNA von Schritt c),
e) Hybridisierung der in Schritt a) erhaltenen Gensequenzen mit der Sonde von Schritt d) und/oder mit der RNA aus Schritt c) und
f) Nachweis einer Veränderung der Expression der Gensequenzen von Schritt a).

Erfindungsgemäß wird hierbei und nachfolgend unter einer pflanzlichen Struktur ein beliebiger Teil einer Pflanze verstanden, d.h. also etwa ein Blatt, Sproßteile, Teile von Wurzeln oder auch Pflanzenzellen, z.B. auch aus der Kultur. Natürlich ist auch jedes Gemisch derartiger Strukturen bei dem erfindungsgemäßen Verfahren einsetzbar.

Beim vorstehend beschriebenen Verfahrensschritt b), der die Behandlung von pflanzlichen Strukturen mit dem auf anti-pflanzenpathogene Wirksamkeit zu prüfenden Agens sowie die Behandlung mit dem Pathogen betrifft, ist die Reihenfolge der Behandlung mit dem Pathogen und mit dem auf anti-pflanzenpathogene Wirksamkeit zu prüfenden Agens gleichgültig; es kann also die pflanzliche Struktur sowohl zuerst mit dem Pathogen und dann mit dem auf anti-pflanzenpathogene Wirksamkeit zu prüfenden Agens behandelt werden, als auch umgekehrt. Die Beliebigkeit der Reihenfolge der Behandlung mit dem Pathogen und mit dem auf anti-pflanzenpathogene Wirksamkeit zu prüfenden Agens gilt auch für alle übrigen Varianten des erfindungsgemäßen Verfahrens.

Unter Hybridisierungssonde wird jegliche Nukleinsäurestruktur verstanden, die mit den Gensequenzen von Verfahrensschritt a) zu hybridisieren in der Lage ist. Wie diese Sonde hergestellt wird, ist primär nicht entscheidend. Es kann sich dabei also um eine chemisch synthetisierte oder aber auch um eine mittels moderner molekularbiologischer Methoden hergestellte Nukleinsäure-Sequenz handeln, insbesondere eine cDNA. Auch kann die RNA direkt ohne Modifizierung eingesetzt werden.

Der Hybridisierungsschritt selbst erfolgt unter Bedingungen, die dem Fachmann bekannt sind (vgl. "Molecular cloning, a laboratory manual", 2. Aufl., J. Sambrook, E. F. Fritsch and T. Maniatis, Cold Spring Harbor Laboratory Press, Cold Spring Harbor, New York, 1989).

Der Nachweis einer Veränderung der Expression der Gensequenzen [in Schritt f)] des vorstehend erwähnten Schritts a) kann mit verschiedenen Techniken geführt werden.

Bei einer bevorzugten Ausführungsform des erfindungsgemäßen Verfahrens erfolgt der Nachweis der Veränderung der Expression der Gensequenzen in Schritt f) mittels reverser Northernhybridisierung von DNA-Arrays. Grundsätzlich ist aber auch der Einsatz einer Northem-blot-Hybridisierungstechnik denkbar ebenso wie Nuklease-Protektion oder klassische RNA-Dotblot-Hybridisierungs sowie der direkte Nachweis von RNA mit elektrochemischen Reportersystemen (elektrischer DNA-Chip).

Eine bevorzugte zweite Ausführungsform des Verfahrens gemäß dem ersten Aspekt der Erfindung umfaßt die Schritte:
a) Anlegen einer Genbank mit Gensequenzen, deren Expression sich bei Infektion von Pflanzen mit Pathogenen ändert,
b) Behandlung von pflanzlichen Strukturen mit dem auf anti-pflanzenpathogene Wirksamkeit zu prüfenden Agens sowie Behandlung mit dem Pathogen,
c) Isolierung der RNA des Behandlungsansatzes von Schritt b), bevorzugterweise Isolierung der mRNA,
d) Herstellung einer cDNA zu der RNA von Schritt c),
e) Nachweis einer Veränderung der Expression der Gensequenzen von Schritt a).

Bei einer bevorzugten Ausführungsform dieses Verfahrens wird der Nachweis der Veränderung der Expression der Gensequenzen, d.h. ob sich die Menge und/oder die Zusammensetzung der in Schritt c) isolierten RNA geändert hat, in Schritt e) mittels einer PCR (polymerase-chain-reaction) unter Einsatz entsprechender Primer durchgeführt (Methode der RT-PCR). Dazu wird die zu untersuchende RNA-Population mit Hilfe der reversen Transkriptase (RT) und ensprechender Oligonukleotidprimer in cDNA umgeschrieben. Die Anwesenheit bzw. die Konzentration einer cDNA für ein Gen von Interesse kann dann mit Hilfe der PCR-Reaktion mit den genspezifischen Oligonukleotidprimern und den bei der PCR-Reaktion üblichen Nachweisverfahren bestimmt werden.

Erfindungsgemäß erfolgt bei den verschiedenen Aspekten des erfindungsgemäßen Verfahrens das Anlegen der Genbank - in Schritt a) - vorzugsweise, indem
a-1) pflanzliche Strukturen mit dem Pathogen behandelt werden,
a-2) mRNA aus den Strukturen von a-1), aus nicht-behandelten pflanzlichen Strukturen und aus dem Pathogen isoliert wird,
a-3) cDNA der in Schritt a-2) erhaltenen mRNA hergestellt wird,
a-4) aus der cDNA von Schritt a-3) unter Einsatz von subtraktiver Hybridisierung und Suppressions-PCR die Genbank angelegt wird.

Gemäß dem zweiten Aspekt der Erfindung wird erfindungsgemäß zur Lösung der Aufgabe ein Verfahren bereitgestellt, bei dem die Erkennung der Wirksamkeit anhand der Genexpression auf Promotorebene erfolgt.

Bei diesem zweiten Aspekt der Erfindung umfaßt eine bevorzugte Ausführungsform die Schritte:
a) Isolierung der Promotor-Sequenzen von Genen, deren Aktivität sich bei infektion von Pflanzen mit Pathogenen ändert.
b) Herstellung eines Reporter-Konstrukts, enthaltend den Promotor und ein Reportergen,
c) Einbringen des Konstrukts in eine pflanzliche Struktur,
d) Behandlung der pflanzlichen Struktur mit dem auf antipflanzenpathogene Wirksamkeit zu prüfenden Agens sowie Behandlung mit dem Pathogen,
e) Nachweis einer Veränderung der Expression des Reporter-Konstrukts in den behandelten pflanzlichen Strukturen.

Auch hier ist die Reihenfolge der Behandlung mit dem auf anti-pflanzenpathogene Wirksamkeit zu prüfenden Agens und mit dem Pathogen gleichgültig.

Unter Reportergenen werden solche Gene verstanden, deren Genprodukte Enzyme darstellen, welche biochemische Reaktionen katalysieren, bei denen gefärbte bzw. fluoreszierende Substanzen entstehen, die sehr leicht in den transgenen Zellgeweben nachgewiesen werden können. Die Stärke der Farb- bzw. Fluoreszenzreaktion ist dabei ein Maß für die Menge an synthetisiertem Reporter-Enzym. Für das erfindungsgemäße Verfahren sind dabei insbesondere die β-Glucuronidase, die Luciferase oder das "Green Fluorescent Protein" (GFP) von Bedeutung.

Der Begriff "Konstrukt" bedeutet hier und nachfolgend, daß darunter alle chimären Gene zu verstehen sind, die am 5'-Ende aus dem aus Schritt a) isolierten Promotorfragment, zentral aus einem der erwähnten Reportersequenzen und 3'aus einer Terminatorsequenz, wie z.B. dem NOS-Terminator, zusammengesetzt sind. Das chimäre Gen ist dabei in ein gängiges Pflanzen-Transformations-Plasmid ligiert, welches Wachstum und Selektion des Konstrukts in *E*.*coli* -Zellen ermöglicht und zum anderen den pflanzlichen Selektionsmarker trägt.

Das Einbringen der Konstrukte kann mit Standard-Techniken erfolgen wie z.B. mit Hilfe von Agrobakterien oder durch direkten DNA-Transfer mittels Mikropartiketbombardierung von Pflanzengewebe oder Elektroporation oder Polyethylenglycol(PEG)-Behandlung von pflanzlichen Protoplasten. Die verschiedenen Methoden sind in dem Buch "Practical Application of Plant Molecular Biology" (Henry 1997, Chapman & Hall, London) zusammenfassend beschrieben.

Bei den in den erfindungsgemäßen Verfahren eingesetzten Pathogenen handelt es sich vorzugsweise um Insekten, Viren, Bakterien, Pilze oder Nematoden. Besonders erwähnt seien dabei die Pathogene, die im einzelnen in den bekannten Phytomedizinbüchern aufgeführt sind (z.B. "Parasitäre Krankheiten und Schädlinge an landwirtschaftlichen Kulturpflanzen", G.M. Hoffmann & H. Schmutterer, Hrsgg., Verlag Eugen Ulmer, Stuttgart, 1983 und "Krankheiten der Wald- und Parkbäume", H. Butin, Hrsg., Georg Thieme Verlag Stuttgart-New York, 1996).

Als Agens kommen für den Einsatz bei dem erfindungsgemäßen Verfahren in allen seinen Aspekten grundsätzlich alle chemisch synthetisierten und/oder natürlichen Substanzen in Frage. Diese können eine anti-pflanzen-pathogene Wirksamkeit, insbesondere fungizide, insektizide, bakterizide, virotoxische oder namatotoxische Wirksamkeit besitzen.

Die bei dem erfindungsgemäßen Verfahren eingesetzten Gensequenzen sind aus Gensequenzen ausgewählt, die bei Infektion von Pflanzen mit Pathogenen differentiell exprimiert werden, d.h. ihre Expression ist geändert, was einerseits bedeuten kann, daß die Expression entweder induziert oder gegenüber einem Basiswert verstärkt wird, andererseits aber auch, daß sie reduziert werden kann.

Bei diesen Gensequenzen handelt es sich insbesondere um solche der pflanzlichen Abwehrantwort, bevorzugt um ein Abwehrgen, ein wundinduziertes Gen oder deren Homologe. Hierbei und auch sonst im Verlaufe der Beschreibung der Erfindung ist "ein" immer im Sinne von "irgendein", d.h. auch beliebig mehrere, zu verstehen, sofern nichts anderes angegeben ist.

Unter homologen Genen werden im Rahmen der Erfindung Gene verstanden, welche in verschiedenen Organismen identische Funktionen ausüben; insbesondere sind solche Gene gemeint, die bei einer Hybridisierung unter bestimmten Bedingungen, etwa (30 % (v/v) Formamid, 6 x SSC, 5 x Denhardts, 0,1 % SDS, bei 42 °C; gewaschen mit 2 x SSC bei 65°C) in der Lage sind, miteinander zu hybridisieren.

Zu den Genen der pflanzlichen Abwehrantwort gehören insbesondere (zusammengefasst in den Lehrbüchern "Phytopathologie - Allgemeine und biochemische Grundlagen", E.F. Elstner, W. Oswald und I. Schneider, Hrsgg., Spektrum Akademischer Verlag Heidelberg, 1996, und "Plant Pathology", 4. Aufl., G.N. Agrios Ed., Academic Press San Diego, 1997): Abwehrgene, insbesondere die "Pathogenesis Related" Gene (PR-Gene) [Kombrink und Somssich. "The Mycota V, Part A, Plant Relationships", Carroll/Tudzynski (Hrsg.), S.107 bis 128, Springer Verlag Berlin Heidelberg New York (1997)], Gene der "Systemic Acquired Resistance" (SAR-Gene) wie z.B. das Gene des Prosystemins oder Proteinaseinhibitoren-Gene (pin1 und 2), Gene des Hypersensitiven Respons (HR-Gene) wie etwa das Gen der ACC-Synthetase, ACC-Oxidase oder der NADH-Oxidase, Gene für die Papillenbildung wie etwa der hydoxyprolinreichen Glykoproteine (HPRG) oder der glycin-prolinreichen Proteine, wundinduzierte Gene, stressinduzierte Gene wie Lipoxygenase, Katalase, Glutathion S-Transferase (GST) oder Enzyme des Phenol- und Phytoalexin-Stoffwechsels wie Phenylalaninammoniumlyase (PAL), Chalconsynthase (CHS), 4-Cumaryl-CoA-Ligase (4CL), Stilbensynthetase (STS), Peroxidasen (POD), Polyphenoloxidasen (PPO). Erfindungsgemäß kann es sich bei der differentiell exprimierten Gensequenz auch um ein *in planta* induziertes (ipi) Gen des Pathogens handeln, wie z.B. das ipiO-Gen (West et al. 1998, Fungal Genet. Biol. 23, 126-138), Avirulenz-Gene (avr/pth), hpr-Gene (Gene für HR und Pathogenität), Gene für Pathogenitäts-Faktoren (pat) oder "disease-specific" Gene (dsp), Gene für zellwandabbauende Enzyme wie pectolytische Enzyme, Cutinasen oder Cellulasen (zusammengefasst in "Plant Pathology", 4. Aufl., G.N. Agrios Hrsg., Academic Press San Diego, 1997).

Erfindungsgemäß werden die Gene der vorstehend erläuterten pflanzlichen Abwehrantwort oder die *in planta* induzierten Gene eines Pathogens, insbesondere die vorstehend genannten wundinduzierten Gene, stressinduzierten Gene sowie die in der nachfolgenden Tab. I aufgelisteten Gene oder Homologe der genannten Gene zur Erkennung und/oder Charakterisierung der Wirksamkeit anti-pflanzenpathogener Agenzien, insbesondere zum Screening anti-pflanzenpathogener Agenzien verwendet.

Das erfindungsgemäße Verfahren in allen seinen Varianten eignet sich prinzipiell für alle Pflanzen bzw. deren Gene. Zu diesen Pflanzen gehören insbesondere Pflanzen, die in Deutschland angebaut werden. Dazu gehören Pflanzen des Ackerbaus (Getreide einschließlich Mais, Kartoffel, Zukker- und Futterrübe, Raps und Rübsen, Klee, Lupine, Luzerne, Tabak, Hopfen sowie Pflanzen aus der Grünlandbewirtschaftung). Pflanzen des Gemüsebaus (Erbse, Bohne, Gurke, Tomate, Kohl und Kohlrübe, Rettich und Radieschen, Möhre, Salat und Endivie, Sellerie, Zwiebel und Lauch, Spargel), Pflanzen des Obstbaus (Apfel, Birne, Kirsche, Pflaumen und Zwetschge, Pfirsich, Erdbeere, Johannisbeere, Stachelbeere, Himbeere, Weinrebe), ebenso forstwirtschaftliche Pflanzen (Fichte, Kiefer, Tanne, Lärche, Douglasie, Eiche, Rotbuche, Ahorn, Esche, Ulme, Vogelkirsche, Linde, Hainbuche, Birke, Pappel, Aspe, Rosskastanie, Robinie, Weide), aber auch alle wirtschaftlich genutzten Pflanzen, die in Deutschland nicht angebaut werden können. Dazu gehören insbesondere die Pflanzen die in dem vorstehend erwähnten Lehrbuch "Plant Pathology" (G.N. Agrios, Hrsg.) aufgeführt sind.

Die Isolierung bzw. Herstellung der in Tab. 1 wiedergegebenen DNA-Sequenzen erfolgt mit einem Verfahren, umfassend die Schritte
a) Behandlung von pflanzlichen Strukturen von Kartoffeln mit dem Pathogen *Phytophthora infestans,*
b) Isolierung der mRNA aus den Strukturen von a), aus nicht-behandelten pflanzlichen Strukturen und aus dem Pathogen,
c) Herstellung der cDNA von der in Schritt b) erhaltenen mRNA,
d) Anlegen einer Genbank aus der cDNA von Schritt c) unter Einsatz von subtraktiver Hybridisierung und Suppressions-PCR.

Im einzelnen sind die vorstehend genannten Verfahrensschritte, die bei der Isolierung der Kartoffel- bzw. *Phytophthora infestans*-DNA-Sequenzen durchgeführt wurden, in den Erläuterungen zu dem erfindungsgemäßen Verfahren, insbesondere Screening-Verfahren, zur Erkennung und/oder Charakterisierung der anti-pflanzenpathogenen Wirksamkeit von Agenzien, wobei die Erkennung bzw. die Charakterisierung der Wirksamkeit anhand der Veränderung der Expressionsstärke der Gene auf molekularer Ebene bei Infektion von Pflanzen mit Pathogenen erfolgt, näher ausgeführt.

Die Erfindung wird nachfolgend durch die Beispiele und die beigefügten Fig. näher erläutert. Es zeigen die Fig.:
Fig. 1 zeigt das Ergebnis des Anwendungsbeispiels 1, bei dem die in den Herstellungsbeispielen 1 und 2 erzeugte Genbank nach differentiell exprimierten Genen untersucht wurde.
Fig. 2 zeigt das Ergebnis des Anwendungsbeispiels 2, bei dem die Wirksamkeit eines Fungizids mit Hilfe differentiell exprimierter Gene und DNA-Array-Hybridisierungstechnik sichtbar gemacht ist.
Die Abkürzungen M oder mM stehen im Folgenden für die Einheit mol/l bzw. mMol/l.

### Beispiele

Sichtbarmachung der fungiziden Wirksamkeit einer Substanz bei der Infektion von Kartoffelblättern mit einer Zoosporensupension des Oomyceten *Phytophthora infestans* (Erreger der Krautfäule an Kartoffel und Tomate)

Bei den Beispielen wurden zunächst (Herstellungsbeispiel 1) cDNA-Fragmente von Genen isoliert, welche ausschließlich bzw. verstärkt in Phytophthora-infizierten Kartoffelblättern exprimiert werden (differentiell exprimierte Gene sowohl der Pflanze als auch des Erregers).

Anschließend wurden Proben der differentiell exprimierten Gene auf Membranfilter gitterförmig aufgetragen (Herstellungsbeispiel 2), wodurch sog. cDNA-Filter-Arrays entstanden. Für rund die Hälfte der gedotteten Gene konnte mit Hilfe von "reverser Northernhybridisierung" differentielle Expression in infizierten Blättern nachgewiesen werden (Anwendungsbeispiel 1). Die cDNA-Arrays wurden anschließend dazu benutzt, die fungizide Wirksamkeit einer Substanz (Metalaxyl) zu erkennen (Anwendungsbeispiel 2).

### Herstellungsbeispiel 1

### Klonierung von Genen, deren Expression durch die Infektion induziert wird, mit Hilfe von cDNA-Subtraktion und "Suppressions-PCR" (Gen-Identifizierung)

Zur Identifizierung der Gene, welche ausschließlich bzw. verstärkt im infiziertem Gewebe exprimiert werden (sowohl Pflanzengene als auch Gene des Erregers), wurde die Methode der subtraktiven Hybridisierung gekoppelt mit Suppressions-PCR angewandt. Das Experiment wurde mit dem "CLONTECH PCR-Select^{™} cDNA Subtraction Kit" der Fa. CLONTECH (www.clontech.com) (U.S. Patent #5,565,340) (Katalog-Nr. K1804-1) exakt nach Angaben des Herstellers durchgeführt. Die Methode ist datailliert von Ulrich Thoenes in Biospektrum 2. Jahrgang, 6.96, S.51-52, beschrieben. Sie wird in der Literatur auch als "Suppression Subtractive Hybridization" (SSH) bezeichnet (Diatchenko L. et al., 1996, Proc. Natl. Acad. Sci. USA 93, 6025-6030). Sie dient dazu, in cDNA umgeschriebene mRNA-Populationen miteinander zu vergleichen und differentiell exprimierte Gene, die nur in einer mRNA-Population vertreten sind, selektiv anzureichern, wodurch die entsprechenden cDNAs isoliert und charakterisiert werden können.

In dem beispielsgemäßen Verfahren wurde die mRNA-Population von infiziertem Blattmaterial ("natürliche" Mischung aus Wirt- und ErrergermRNA) mit der mRNA-Population, bestehend aus einer "künstlichen" Mischung aus mRNA von nicht infizierten Blätter und mRNA vom kultivierten Erreger, miteinander verglichen. Dabei "eliminieren" sich die RNAs, welche beiden RNA-Pools angehören, gegenseitig. Nur die spezifisch im infizierten Blattmaterial gebildeten mRNAs (in Form der umgeschriebenen cDNAs) bleiben übrig.

### mRNA-Isolation:

Hierzu wurden Kartoffelblätter mit einer Suspension von *Phytophthora infestans* Zoosporen infiziert. Kontrollblätter wurden nur mit Wasser behandelt. Anschließend wurden die behandelten Blätter für drei Tage in einer Klimakammer inkubiert. Nach der Inkubation wurde aus den Blättern die Gesamt-RNA präpariert. Ebenso wurde die Gesamt-RNA aus einer *Phytophthora*-Kultur gewonnen, die auf einem Nähragar aus Gemüsesaft gewachsen war. Die Methode der RNA-Isolation ist bei Pasentsis et al. [The Plant Journal (1998) 13 (1), 51-61] genau beschrieben. Bei der RNA-Präparation aus infizierten Blättern handelt es sich um eine Mischung aus Wirtspflanzen-RNA und RNA des Erregers, welcher sich in der Wirtspflanze vermehrt hat. Aus den Gesamt-RNAs wurden anschließend die entsprechenden mRNAs isoliert. Dies geschah durch Chromatographie der Gesamt-RNAs an OligodT-Zellulose mit Hilfe von kleinen Spinsäulchen der Fa. Pharmacia (www.apbiotech.com) (Produkt-Nr. 27-9258-01) laut Firmenanleitung. Entsprechend der Zusammensetzung der Gesamt-RNA bestand die mRNA-Präparation aus den infizierten Blättern, wie bereits erwähnt, aus einer Mischung von mRNA der Wirtsplanze und mRNA des Erregers. Ebenso wurde die mRNA aus der Gesamt-RNA isoliert, welche aus der *Phytophthora*-Kultur gewonnen worden war.

### cDNA-Synthese, Subtraktive Hybridisierung und Suppressions-PCR

Dabei wurde genau nach Kit-Vorschrift verfahren, wobei die oben beschriebenen mRNA-Präparationen in den Versuch eingesetzt wurden. Der eine Ansatz (in der Kitanleitung als "Tester" bezeichnet) enthielt die "künstliche" Mischung aus mRNA der Kontrollblätter und der *Phytophthora*-Kultur, der andere Ansatz (in der Kitanleitung als "Driver") enthielt die mRNA der mit Zoosporen behandelten Blätter. Die Driver-mRNA-Population enthält die mRNAs, deren Expression spezifisch durch die Infektion induziert wird. Tester- und Driver-mRNA wurden mit Hilfe des modifizierten Oligo-dT-cDNA-Syntheseprimers (Kit-Bestandteil) in cDNA umgeschrieben. Die synthetisierten cDNA-Populationen wurden mit Hilfe der subtraktiven Hybridisierung miteinander verglichen und die im infizierten System differentiell exprimierten Gene (Pflanze und Pilz) wurden anschließenden mit Suppressions-PCR (Diatchenko L. et al., 1996, Proc. Natl. Acad. Sci. USA 93, 6025-6030) spezfisch vermehrt.

### Klonierung der Genfragmente, Herstellung einer Genbank

Die PCR-Fragmente der differentiell exprimierten Gene wurden dann mit Hilfe des TA-Klonierungsystems der Fa. INVITROGEN (www.clontech.com) (U.S. Patent #5,487,993) (Katalog-Nr. K2030-01) in den Vektor pCR1.2 ligiert und der Ligationsansatz wurde in competente *E*.*coli-*Zellen TOP10F' (Fa. INVITROGEN) transformiert. Aus der entstandenen Genbank, die rund 100.000 primäre Transformanten enthält und die angereichert ist auf Gene, die infektionsabhängig exprimiert werden, wurden 96 Klone zufällig herausgepickt. Mittels Kolonie-PCR wurde aus den entsprechenden Bakterienkolonien die klonierten Genfragmente herausamplifiziert. Die durch die PCR-Reaktionen entstandenen doppelsträngigen DNA-Fragmente wurden mit Hilfe von Natronlauge (Endkonzentration = 0,2 M) zur Einzelstrangform denaturiert.

### Herstellungsbeispiel 2: Anlegen von cDNA-Arrays

Aliquots der im Herstellungsbeispiel 1 erhaltenen einzelsträngigen DNA-Proben wurden dann gitterförmig im Muster von 96-Well Mikrotiterplatten auf Membranfilter gedottet (cDNA-Array), wobei der Abstand der einzelnen Dots zueinander nach oben, nach unten und zu beiden Seiten je 4,5 mm betrug.

### Anwendungsbeispiel 1

### Sichtung der Genbank nach differentiell exprimierten Genen:

Die benutzte Technik wird als reverse "Northernhybridisierung" oder auch als "Multiplex Messanger Assay" (MMA) bezeichnet. Dazu werden identische cDNA-Arrays mit komplexen Proben hybridisiert, die durch die Umschreibung von mRNA in markierte cDNA unter Verwendung eines OligodT-Primers hergestellt werden. Ein entsprechender Versuch ist im Zusammenhang mit der T-Zellen-Aktivierung von Karine Bernard et al. (Nucleic Acids Research, 1996, Vol. 24, No. 8, 1435-1442) beschrieben worden.

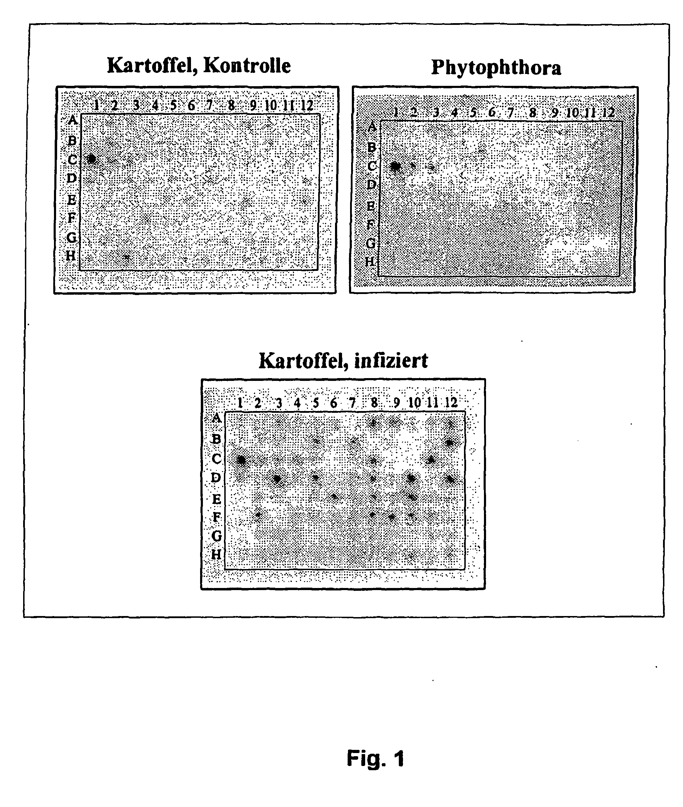

Es wurden Proben aus der RNA von Kontrollpflanzen, *Phytophthora-*infizierten Pflanzen und von *Phytophthora-*Kulturen hergestellt. Die Gegenwart einer mRNA in den RNA-Präparationen (exprimiertes Gen), für welche ein Gen kodiert, von dem ein DNA-Stück auf den Filtern gedottet ist, wird durch ein Signal auf den Filtern erkennbar (schwarze Punkte in Fig. 1). Die Größe eines Dots (Signal) und dessen Schwärzungsgrad ist ein Maß für die Menge der entsprechenden mRNA in der RNA-Probe und damit ein Maß für den Expressionsgrad des entsprechenden Gens. Die Signale (Dots) können mit entsprechenden Geräten (Scanner; Phosphorimager) quantifiziert werden. Zur besseren Quantifizierung der Signale wurde den RNA-Präparationen ein selbst hergestelltes Transkript, welches wie die nativen mRNAs einen längeren 5'-terminalen Poly-A-Schwanz trägt, als interner Standard in definierter Konzentration beigemischt. Die Menge des Transkripts korreliert mit der Signalstärke an Position C1 des DNA-Arrays (Fig. 1), an dem sich eine DNA-Probe für das Transkript befindet. Die Stärke der anderen Signale auf dem Array kann dann in Relation zu dem Signal auf Position C1 angegeben werden.

Bei der Probenherstellung wurde wie folgt vorgegangen: 10 µg der entsprechenden Gesamt-RNA-Präparation wurden mit einer definierten Mange Kontroll-Transkript versetzt und dem Ansatz 1 µg Oligo-dT₁₂₋₁₈ der Fa. Pharmacia (www.apbiotech.com) (Produkt-Nr. 27-7878-01) zugegeben. Das Reaktionsvolumen wurde mit sterilem Wasser auf 11 µl eingestellt und der Ansatz zur Denaturierung der RNA für 10 Minuten bei 65 °C inkubiert. In der Zwischenzeit wurde ein Reaktionsmix mit folgender Zusammensetzung hergestellt:

| Reaktionsmix | |
|---|---|
| 5 x RT-Puffer | 5 µl |
| Gibco/BRL | |
| | |
| 0,1 MDTT | 2,5 µl |
| | |
| dCTP (0,1 mM) | 1 ,25 µl |
| | |
| DATP / dTTP / dGTP-Mix | 1,25 µl |
| (jeweils 10 mM) | |
| | |
| RNAguard (Pharmacia) | 1 µl |
| Nr. 27-0815-01; 29,4 U/µl | |
| | |
| SuperScript II (Gibco/BRL, | 1 µl |
| Nr. 18064-014; 200 U/µl) | |
| | |
| [α- ³²P]dCTP | 2 bis 5 µl |
| (Amersham, 3000 | |
| Ci/mmol; 10 µCi/µl) | |

Nach der Denaturierung wurde die RNA-Probe auf 42 °C abgekühlt und mit auf 42 °C vorgewärmten Reaktionsmix gemischt. Die Synthese der ³²P-markierten cDNA fand dann bei 42 °C eine Stunde lang statt. Nicht eingebautes ³²P-markiertes dCTP wurde von der Probe durch Gelfiltration an einer Sephadex G50-Minisäule (Pharmacia) (www.apbiotech.com) (Produkt-Nr. 27-5330-01) abgetrennt. Für die markierten Proben wurde eine radioaktive Aktivität von rund 1- 50 × 10⁶ dpm gemessen. Die cDNA-Array-Filter wurden mit Hybridisierungslösung [1 % (w/v) Blocking-Reagenz (Boehringer Mannheim) (biochem.boehringer-mannheim.com) (Produkt-Nr. 1096176), 2 x SSC, 1 % (w/v) N-Laurylsarcosinat, 1 % (w/v) SDS] bei 65 °C für 1 bis 4 Stunden vorhybridisiert. Anschließend wurde die Hybridisierungslösung abgeschüttet und durch 65 °C warme Hybridisierlösung ersetzt, die rund 5 x 10⁸ dpm radioaktive cDNA-Probe enthielt. Die Hybridisierung wurde dann für rund 16 Stunden bei 65 °C durchgeführt. Nach der Hybridisierung wurden die Filter zweimal für rund 20 Minuten mit 2 x SSC, 0,1 % (w/v) SDS und dreimal für 20 Minuten mit 0,2 x SSC, 0,1 % SDS (w/v) bei 65 °C gewaschen. Danach wurden die Filter kurz getrochnet und mit Hilfe des STROM Phosphor-Imagers (Molecular Dynamics, USA) (www.mdyn.com) ausgewertet. Die Ergebnisse sind in Fig. 1 gezeigt.

Wie aus Fig. 1 ersichtlich, liefern eine ganze Reihe von Genen (Dots) mit der Probe, die aus RNA von infizierten Kartoffelblättern hergestellt wurde, Signale. Die meisten dieser Gene reagieren nicht mit Proben, die mit RNAs aus Kontrollblättern oder aus Phytophtora-Kulturen hergestellt wurden. Diese Gene werden also nur bei Infektion exprimiert (differentiell exprimierte Gene). Deren Expressionsgrad stellt eine sowohl qualitativ als auch quantitativ bestimmbare Kenngröße für den Verlauf der Infektion dar.

### Ausführungsbeispiel 2

### Sichtbarmachung der Wirksamkeit eines Fungizids mit Hilfe der oben beschriebenen differentiell exprimierten Gene und DNA-Array-Hybridisierungstechnik

Grundlage des Nachweises ist die Tatsache, daß die Expression der infektions-induzierten Gene durch die Metalaxyl-Behandlung aufgrund der fungiziden Wirkung der Substanz deutlich reduziert wird. Die Wirksamkeit kann folglich daran erkannt werden, daß die Signale auf den cDNA-Arrays nach Hybridisierung mit einer Probe aus wirkstoffbehandelten Blattmaterial, verglichen mit der Probe aus unbehandelten Blättern, abgeschwächt sind.

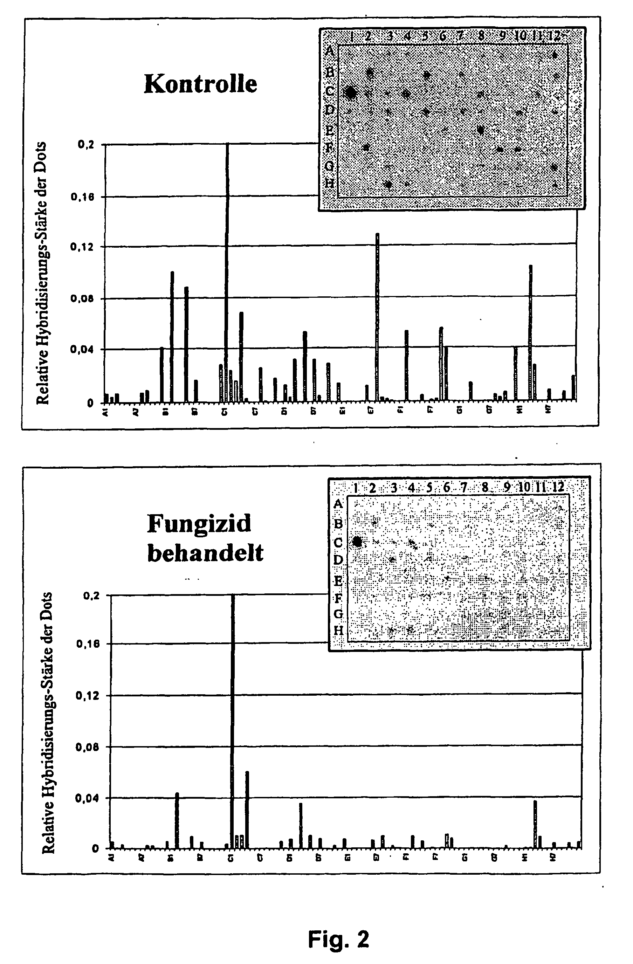

Es wurden Kartoffelblätter mit einer Lösung einer fungizid wirksamen Substanz (Metalaxyl) in einer Konzentration von 250 und 500 ppm behandelt und in einer Klimakammer für 24 Stunden inkubiert. Zur Kontrolle wurden Blätter derselben Kartoffelpflanze nur mit Wasser behandelt und in gleicher Weise wie die Testblätter weiterbehandelt. Die fungizidbehandelten Blätter und die Kontrollblätter wurden anschließend mit *Phytophthora* Zoosporen infiziert und für weitere 12 bis 72 Stunden in einer Klimakammer inkubiert. Nach der Inkubation wurde aus den Blättern die Gesamt-RNA präpariert und aus diesen cDNA-Hybridisierungsproben wie oben beschrieben synthetisiert. Identische, wie oben beschriebene cDNA-Arrays wurden mit Proben aus infizierten Blättern, welche mit dem Fungizid bzw. mit Wasser vorbehandelt worden waren, hybridisiert. Die Ergebnisse sind in Fig. 2 gezeigt.

Wie in Fig. 2 ersichtlich, ist die Intensität der Signale mit der Probe 'fungizidbehandelte Blätter' gegenüber der Intensität der Signale mit der Probe 'wasserbehandelte Blätter' (Kontrolle) deutlich reduziert. Dies bedeutet, daß die infektionsinduzierten Gene infolge der Fungizidbehandlung wesentlich schwächer exprimiert werden als in der Kontrollpflanze. Damit ist der Expressionsgrad der Gene ein Maß für die Wirksamkeit des applizierten Fungizids, wobei sich der Expressionsgrad der Gene in der Signalstärke der entsprechenden Dots auf dem DNA-Array widerspiegelt.

### SEQUENZPROTOKOLL

### ALLGEMEINE ANGABEN

ANMELDER
   NAME: FELSENSTEIN, FRIEDRICH
   STRASSE: RINGSTRASSE 30
   ORT: ATTENKIRCHEN
   BUNDESLAND: BAYERN
   LAND: DEUTSCHLAND
   POSTLEITZAHL: 85395
NAME: THUEMMLER, FRITZ
   STRASSE: AM BERGMOOS 6A
   ORT: KIRCHDORF
   BUNDESLAND: BAYERN
   LAND: DEUTSCHLAND
   POSTLEITZAHL: 85414
BEZEICHNUNG DER ERFINDUNG: VERFAHREN ZUR ERKENNUNG UND CHARAKTERISIERUNG VON WIRKSTOFFEN GEGEN PFLANZEN-PATHOGENE
ANZAHL DER SEQUENZEN: 20
   SONSTIGE ANGABEN: N bedeutet beliebiges von A,C,G,T
   COMPUTERLESBARE FASSUNG:
   DATENTRAEGER: DISKETTE 1,44 MB HD
   COMPUTER: IBM PC-KOMPATIBEL
   BETRIEBSSYSTEM: MS-DOS 6.20
   SOFTWARE: WORD FUER DOS 4.0
DATEN DER JETZIGEN ANMELDUNG
   ANMELDENUMMER: 198 60 313.4
   ANMELDETAG: 24.12.98
   KLASSIFIKATION: C12N
ANGABEN ZUM VERTRETER:
   NAME: KLOSE, ULRICH
   VERTRETERNUMMER: 291013
   AKTENZEICHEN: FS9801P
TELEKOMMUNIKATION:
   TELEFON: 08142-58326
   TELEFAX: 08142-540876
   ELEKTRONISCHE POST: CHEMPAT.KLOSE@t-ONLINE.DE
ANGABEN ZU SEQ ID-NO:1:
SEQUENZCHARAKTERISTIKA:
   LAENGE: 276 BASENPAARE
   ART: NUKLEINSAEURE
   STRANGFORM: DOPPELSTRANG
   TOPOLOGIE: LINEAR
ART DES MOLEKUELS: c-DNA zu m-RNA
URSPRUENGLICHE HERKUNFT: SOLANUM TUBEROSUM
SEQUENZBESCHREIBUNG: SEQ ID-NO:1:
ANGABEN ZU SEQ ID-NO:2:
SEQUENZCHARAKTERISTIKA:
   LAENGE: 492 BASENPAARE
   ART: NUKLEINSAEURE
   STRANGFORM: DOPPELSTRANG
   TOPOLOGIE: LINEAR
ART DES MOLEKUELS: c-DNA zu m-RNA
URSPRUENGLICHE HERKUNFT: SOLANUM TUBEROSUM
SEQUENZBESCHREIBUNG: SEQ ID-NO:2:
ANGABEN ZU SEQ ID-NO:3:
SEQUENZCHARAKTERISTIKA:
   LAENGE: 678 BASENPAARE
   ART:·NUKLEINSAEURE
   STRANGFORM:·DOPPELSTRANG
   TOPOLOGIE:·LINEAR
ART·DES·MOLEKUELS:·c-DKA·zu·m-RNA
URSPRUENGLICHE · HERKUNFT: · SOLANUM·TUBEROSUM
SEQUENZBESCHREIBUNG:·SEQ·ID-NO:3:
ANGABEN ZU SEQ ID-NO:4:
SEQUENZCHARAKTERISTIKA:
   LAENGE: 691 BASENPAARE
   ART:·NUKLEINSAEURE
   STRANGFORM:·DOPPELSTRANG
   TOPOLOGIE:·LINEAR
ART·DES·MOLEKUELS:·c-D'NA·zu·m-RNA
URSPRUENGLICHE·BERKUNFT:·SOLANUM·TUBEROSUN
SEQUENZBESCHREIBUNG:-SEQeID-HO:4:
ANGABEN ZU SEQ ID-NO:5:
SEQUENZCHARAKTERISTIKA:
   LAENGE: 487 BASENPAARE
   ART: · NUKLEINSAEURE
   STRANGFORM: · DOPPELSTRANG
   TOPOLOGIE:·LINEAR
ART·DES·MOLEKUELS:·c-DNA·zu·m-RNA
URSPRUENGLICHE·HERKUNFT:·SOLANUM·TUBEROSUM
SEQUENZBESCHREIBUNG:·SEQ·ID-NO:5:
ANGABEN ZU SEQ ID-NO:6:
SEQUENZCHARAKTERISTIKA:
   LAENGE: 462 BASENPAARE
   ART:-NUKLEINSAEURE
   STRANGFORM:-DOPPELSTRANG
   TOPOLOGIE:-LINEAR
ART·DES·MOLEKUELS:·c-DNA·zu·m-RNA
URSPRUENGLICHE·HERKUNFT:·SOLANUM·TUBEROSUM
SEQUENZBESCHREIBUNG:-SEQ-ID-NO:6:
ANGABEN ZU SEQ ID-NO:7:
SEQUENZCHARAKTERISTIKA:
   LAENGE: 394 BASENPAARE
   ART:-NUKLEINSAEURE
   STRANGFORM:-DOPPELSTRANG
   TOPOLOGIE:-LINEAR
ART·DES·MOLEKUELS:·c-DNA·zu·m-RNA
URSPRUENGLICHE-HERKUNFT:-SOLANUM-TUBEROSUM
SEQUENZBESCHREIBUNG:-SEQ-ID-NO:7:
ANGABEN ZU SEQ ID-NO:8:
SEQUENZCHARAKTERISTIKA:
   LAENGE: 447 BASENPAARE
   ART:-NUKLEINSAEURE
   STRANGFORM:·DOPPELSTRANG
   TOPOLOGIE:-LINEAR
ART·DES·MOLEKUELS:·c-DNA·zu·m-RNA
URSPRUENGLICHE·HERKUNFT:·SOLANUM·TUBEROSUM
SEQUENZBESCHREIBUNG: .SEQ.ID-NO:8:
ANGABEN ZU SEQ ID-NO:9:
SEQUENZCHARAKTERISTIKA:
   LAENGE: 501 BASENPAARE
   ART:·NUKLEINSAEURE
   STRANGFORM:·DOPPELSTRANG
   TOPOLOGIE:·LINEAR
ART·DES·MOLEKUELS:·c-DNA·zu·m-RNA
URSPRUENGLICHE · HERKUNFT: · SOLANUM · TUBEROSUM
SEQUENZBESCHREIBUNG: · SEQ · ID-NO:9:
ANGABEN ZU SEQ ID-NO:10:
SEQUENZCHARAKTERISTIKA:
   LAENGE: 337 BASENPAARE
   ART:·NUKLEINSAEURE
   STRANGFORM:·DOPPELSTRANG
   TOPOLOGIE:·LINEAR
ART·DES·MOLEKUELS:·c-DNA·zu·m-RNA
URSPRUENGLICHE·HERKUNFT:·SOLANUM·TUBEROSUM
SEQUENZBESCHREIBUNG:·SEQ·ID-NO:10:
ANGABEN ZU SEQ ID-NO:11:
SEQUENZCHARAKTERISTIKA:
   LAENGE: 565 BASENPAARE
   ART:·NUKLEINSAEURE
   STRANGFORM:·DOPPELSTRANG
   TOPOLOGIE:·LINEAR
ART-DES-MOLEKUELS:·c-DNA·zu·m-RNA
URSPRUENGLICHE·HERKUNFT:·SOLANUM·TUBEROSUM
SEQUENZBESCHREIBUNG:·SEQ·ID-NO:11:
ANGABEN ZU SEQ ID-NO:12:
SEQUENZCHARAKTERISTIKA:
   LAENGE: 291 BASENPAARE
   ART: NUKLEINSAEURE
   STRANGFORM: DOPPELSTRANG
   TOPOLOGIE: LINEAR
ART:·NUKLEINSAEURE
   STRANGFORM:·DOPPELSTRANG
   TOPOLOGIE:·LINEAR
ART·DES·MOLEKUELS:·c-DNA·zu·m-RNA
URSPRUENGLICHE·HERKUNFT:·SOLANUM·TUBEROSUM
SEQUENZBESCHREIBUNG:·SEQ·ID-NO:12:
ANGABEN ZU SEQ ID-NO:13:
SEQUENZCHARAKTERISTIKA:
   LAENGE: 555 BASENPAARE
   ART: NUKLEINSAEURE
   STRANGFORM: DOPPELSTRANG
   TOPOLOGIE: LINEAR
ART:-NUKLEINSAEURE
   STRANGFORM:·DOPPELSTRANG
   TOPOLOGIE:·LINEAR
ART · DES · MOLEKUELS: · c-DNA · zu ·m-RNA
URSPRUENGLICHE·HERKUNFT:·SOLANUM·TUBEROSUM
SEQUENZBESCHREIBUNG:-SEQ-ID-NO:13:
ANGABEN ZU SEQ ID-NO:14:
SEQUENZCHARAKTERISTIKA:
   LAENGE: 371 BASENPAARE
   ART:·NUKLEINSAEURE
   STRANGFORM:·DOPPELSTRANG
   TOPOLOGIE:·LINEAR
ART·DES·MOLEKUELS:·c-DNA·zu·m-RNA
URSPRUENGLICHE·HERKUNFT:·SOLANUM·TUBEROSUM
SEQUENZBESCHREIBUNG:·SEQ·ID-NO:14:
ANGABEN ZU SEQ ID-NO:15:
SEQUENZCHARAKTERISTIKA:
   LAENGE: 296 BASENPAARE
   ART: ·NUKLEINSAEURE
   STRANGFORM: ·DOPPELSTRANG
   TOPOLOGIE: · LINEAR
ART · DES · MOLEKUELS: · c-DNA ·zu · m-RNA
URSPRUENGLICHE · HERKUNFT: ·SOLANUM · TUBEROSUM
SEQUENZBESCHREIBUNG: SEQ ID-NO:15:
ANGABEN ZU SEQ ID-NO:16:
SEQUENZCHARAKTERISTIKA:
   LAENGE: 432 BASENPAARE
   ART:-NUKLEINSAEURE
   STRANGFORM:·DOPPELSTRANG
   TOPOLOGIE:·LINEAR
ART·DES·MOLEKUELS:·c-DNA·zu·m-RNA
URSPRUENGLICHE-BERKUNFT:-SOLANUM-TUBEROSUM
SEQUENZBESCHREIBUNG:·SEQ·ID-NO:16:
ANGABEN ZU SEQ ID-NO:17:
SEQUENZCHARAKTERISTIKA:
   LAENGE: 297 BASENPAARE
   ART:-NUKLEINSAEURE
   STRANGFORM:-DOPPELSTRANG
   TOPOLOGIE:-LINEAR
ART-DES-MOLEKUELS:-c-DNA-zu-m-RNA
URSPRUENGLICHE-HERKUNFT:-SOLANUM-TUBEROSUM
SEQUENZBESCHREIBUNG:-SEQ-ID-NO:17:
ANGABEN ZU SEQ ID-NO:18:
SEQUENZCHARAKTERISTIKA:
   LAENGE: 556 BASENPAARE
   ART:·NUKLEINSAEURE
   STRANGFORM:·DOPPELSTRANG
   TOPOLOGIE:·LINEAR
ART·DES·MOLEKUELS:·c-DNA·zu·m-RNA
URSPRUENGLICHE·HERKUNFT:·SOLANUM·TUBEROSUM
SEQUENZBESCHREIBUNG:·SEQ·ID-NO:18:
ANGABEN ZU SEQ ID-NO:19:
SEQUENZCHARAKTERISTIKA:
   LAENGE: 472 BASENPAARE
   ART: · NUKLEINSAEURE
   STRANGFORM: · DOPPELSTRANG
   TOPOLOGIE: · LINEAR
ART·DES·MOLEKUELS:·c-DNA·zu·m-RNA
URSPRUENGLICHE·HERKUNFT:·SOLANUM·TUBEROSUM
SEQUENZBESCHREIBUNG:·SEQ·ID-NO:19:
ANGABEN ZU SEQ ID-NO:20:
SEQUENZCHARAKTERISTIKA:
   LAENGE: 500 BASENPAARE
   ART:·NUKLEINSAEURE
   STRANGFORM:·DOPPELSTRANG
   TOPOLOGIE:·LINEAR
ART·DES·MOLEKUELS:·c-DNA·zu·m-RNA
URSPRUENGLICHE · HERKUNFT:·SOLANUM·TUBEROSUM
SEQUENZBESCHREIBUNG:·SEQ·ID-NO:20:

### SEQUENZPROTOKOLL

<110> FELSENSTEIN, FRIEDRICH; THUEMMLER, FRITZ
<120> VERFAHREN ZUR ERKENNUNG UND CHARAKTERISIERUNG VON WIRKSTOFFEN GEGEN PFLANZEN-PATHOGENE
<130> FS9801WO
<140> PCT/DE99/04093
   <141> 2000-01-04
<150> DE 198 60 313.4
   <151> 1998-12-24
<160> 38
<170> PatentIn Ver. 2.1
<210> 1
   <211> 276
   <212> DNA
   <213> Solanum tuberosum
<220>
   <221> CDS
   <222> (2) .. (79)
<400> 1
   t gag atg att agg gaa gca gat gtt gat ggt gat gga caa atc aac tat 49
   Glu Met Ile Arg Glu Ala Asp Val Asp Gly Asp Gly Gln Ile Asn Tyr
   1 5 10 15
   gag gag ttt gtt aag gtc atg atg gcc aag taattccacc atcctcttgt 99
   Glu Glu Phe Val Lys Val Met Met Ala Lys
   20 25
   tacatttgaa gtttaggctt gtcaaaaatg tgaaaaaaaa gccaaaaata attcattgga 159
   taggatctta tctagtataa tgtaagtctt accatctcga tgtattgaac cttcattgaa 219
   tgtaatgttt tattgtaggg gtggtgtaat ctctacttat tttcttgaag tttatgt 276
<210> 2
   <211> 26
   <212> PRT
   <213> Solanum tuberosum
<400> 2
   Glu Met Ile Arg Glu Ala Asp Val Asp Gly Asp Gly Gln Ile Asn Tyr
   1 5 10 15
   Glu Glu Phe Val Lys Val Met Met Ala Lys
   20 25
<210> 3
   <211> 492
   <212> DNA
   <213> Solanum tuberosum
<220>
   <221> CDS
   <222> (1) .. (333)
<400> 3
   gtc aag att gca tgt ttg gtg gtt ttg tgc atg ttg att aca ttt gca   48
   Val Lys Ile Ala Cys Leu Val Val Leu Cys Met Leu Ile Thr Phe Ala
     1               5                  10                  15
   ccc cat gca gag gca gtg act tgt ggc caa ata cag gtt ggg gtg gcg   96
   Pro His Ala Glu Ala Val Thr Cys Gly Gln Ile Gln Val Gly Val Ala
                    20                  25                  30
   aat tgc ctt cct tat ttg cag aac cgt ggc cca ata gga ggc tgt tgt   144
   Asn Cys Leu Pro Tyr Leu Gln Asn Arg Gly Pro Ile Gly Gly Cys Cys
                35                  40                  45
   ggt gtc att aaa gat ttg ctt aag ctt tgt aaa aca cca cat gag cgt   192
   Gly Val Ile Lys Asp Leu Leu Lys Leu Cys Lys Thr Pro His Glu Arg
            50                  55                  60
   aga aaa tca tgt aga tgt gtc aaa aaa gct gct aat act ata aaa ggc   240
   Arg Lys Ser Cys Arg Cys Val Lys Lys Ala Ala Asn Thr Ile Lys Gly
    65                  70                  75                  80
   att gat ttt ggc aaa gct gct ggt ctc tct gga gtt tgt ggt gtc aag   288
   Ile Asp Phe Gly Lys Ala Ala Gly Leu Ser Gly Val Cys Gly Val Lys
                    85                  90                  95
   att cct ttt gag atc agc ccc tct gtt gac tgc tcc aag gtg aag       333
   Ile Pro Phe Glu Ile Ser Pro Ser Val Asp Cys Ser Lys Val Lys
               100                 105                 110
   tgacgttaat gaaagagatt cattttccat atcataatat atggtagtag cacaatcatg 393
   tggacaagaa taagataggt cgagattgat cctgcttaat cacggatcgt gtccgttttc 453
   tctctttagc ttctttgtgt gtgttttttt tttaatcgt                        492
<210> 4
   <211> 111
   <212> PRT
   <213> Solanum tuberosum
<400> 4
   Val Lys Ile Ala Cys Leu Val Val Leu Cys Met Leu Ile Thr Phe Ala
   1               5                   10                  15
   Pro His Ala Glu Ala Val Thr Cys Gly Gln Ile Gln Val Gly Val Ala
               20                  25                  30
   Asn Cys Leu Pro Tyr Leu Gln Asn Arg Gly Pro Ile Gly Gly Cys Cys
           35                  40                  45
   Gly Val Ile Lys Asp Leu Leu Lys Leu Cys Lys Thr Pro His Glu Arg
       50                  55                  60
   Arg Lys Ser Cys Arg Cys Val Lys Lys Ala Ala Asn Thr Ile Lys Gly
   65                  70                  75                  80
   Ile Asp Phe Gly Lys Ala Ala Gly Leu Ser Gly Val Cys Gly Val Lys
                   85                  90                  95
   Ile Pro Phe Glu Ile Ser Pro Ser Val Asp Cys Ser Lys Val Lys
               100                 105                 110
<210> 5
   <211> 678
   <212> DNA
   <213> Solanum tuberosum
<220>
   <221> CDS
   <222> (55)..(363)
<400> 5
   accaaccaca cataattaag caaaaaaata ataattgaga accaaagttg taag atg    57
                                                                     Met
                                                                       1
   gca gaa gtg aag ttg ctt ggt cta agg tat agt cct ttt agc cat aga    105
   Ala Glu Val Lys Leu Leu Gly Leu Arg Tyr Ser Pro Phe Ser His Arg
                   5                   10                  15
   gtt gaa tgg gct cta aaa att aag gga gtg aaa tat gaa ttt ata gag    153
   Val Glu Trp Ala Leu Lys Ile Lys Gly Val Lys Tyr Glu Phe Ile Glu
           20                  25                  30
   gaa gat tta caa aat aag agc cct tta ctt ctt caa tct aat cca att    201
   Glu Asp Leu Gln Asn Lys Ser Pro Leu Leu Leu Gln Ser Asn Pro Ile
       35                  40                  45
   cac aag aaa att cca gtg tta att cac aat ggc aag tgc att tgt gag    249
   His Lys Lys Ile Pro Val Leu Ile His Asn Gly Lys Cys Ile Cys Glu
    50                  55                  60                  65
   tct atg gtc att ctt gaa tac att gat gag gca ttt gaa ggc cct tcc    297
   Ser Met Val Ile Leu Glu Tyr Ile Asp Glu Ala Phe Glu Gly Pro Ser
                   70                  75                  80
   att ttg cct aaa gac tct tat gat cgc gct tta gcg cga ttt tgg gct    345
   Ile Leu Pro Lys Asp Ser Tyr Asp Arg Ala Leu Ala Arg Phe Trp Ala
                   85                  90                  95
   aaa tac gtc gaa gat aag gtatattgct tttaagttat tccaattgat           393
   Lys Tyr Val Glu Asp Lys
           100
   tgaaaaattt gttttagtta cgttattaca tatactttag gtctcatgct ttttaataat 453
   cttttatnaa attcgactaa gacaacttct cgtatagtca caatactaac atatttgtct 513
   agtanttggt taggaaataa gttatccgaa tattaaattc tgggataagt tatgaatacc 573
   atatttgata gttgaattgg gagataaatt attcntgttt aaaattaata tgatatttga 633
   ttgccattta gaaatactaa ctattttatn tgcntagatt ccctn                 678
<210> 6
   <211> 103
   <212> PRT
   <213> Solanum tuberosum
<400> 6
   Met Ala Glu Val Lys Leu Leu Gly Leu Arg Tyr Ser Pro Phe Ser His
   1               5                   10                  15
   Arg Val Glu Trp Ala Leu Lys Ile Lys Gly Val Lys Tyr Glu Phe Ile
               20                  25                  30
   Glu Glu Asp Leu Gln Asn Lys Ser Pro Leu Leu Leu Gln Ser Asn Pro
           35                  40                  45
   Ile His Lys Lys Ile Pro Val Leu Ile His Asn Gly Lys Cys Ile Cys
       50                  55                  60
   Glu Ser Met Val Ile Leu Glu Tyr Ile Asp Glu Ala Phe Glu Gly Pro
   65                  70                  75                  80
   Ser Ile Leu Pro Lys Asp Ser Tyr Asp Arg Ala Leu Ala Arg Phe Trp
                   85                  90                  95
   Ala Lys Tyr Val Glu Asp Lys
               100
<210> 7
   <211> 691
   <212> DNA
   <213> Solanum tuberosum
<220>
   <221> CDS
   <222> (1) .. (615)
<400> 7
   tac gca cca aag aaa gcc agg gtc caa aca gta aag atg ctc atg tta    48
   Tyr Ala Pro Lys Lys Ala Arg Val Gln Thr Val Lys Met Leu Met Leu
    1               5                  10                  15
   tct gtg gtt ggt gga ttt ggt gca ata atc cta gtt act gaa ttg cta    96
   Ser Val Val Gly Gly Phe Gly Ala Ile Ile Leu Val Thr Glu Leu Leu
               20                  25                  30
   ttc aaa gga gcc att cgt gga caa gtt gtt gga tgg att tgc ctt att    144
   Phe Lys Gly Ala Ile Arg Gly Gln Val Val Gly Trp Ile Cys Leu Ile
           35                  40                  45
   ttc tcc tta tgt gta ttt gtt gca ccc tta ggc att gtg aga aaa gtc    192
   Phe Ser Leu Cys Val Phe Val Ala Pro Leu Gly Ile Val Arg Lys Val
       50                  55                  60
   atc aaa aca aaa agt gtg gaa tac atg cca tta ctt cta tca gtt ttt    240
   Ile Lys Thr Lys Ser Val Glu Tyr Met Pro Leu Leu Leu Ser Val Phe
    65                  70                  75                  80
   ctc aca tta agt gct gtt atg tgg ttc ttc tat ggt ctc ttg ctc aaa    288
   Leu Thr Leu Ser Ala Val Met Trp Phe Phe Tyr Gly Leu Leu Leu Lys
                   85                  90                  95
   gat att aac att gct gcn cca aat gta ttg gga ttc atc ttt ggt ata    336
   Asp Ile Asn Ile Ala Ala Pro Asn Val Leu Gly Phe Ile Phe Gly Ile
               100                 105                 110
   ctc caa att gtg ttg tat gca ata tac agc aaa aaa gag aag gtc att    384
   Leu Gln Ile Val Leu Tyr Ala Ile Tyr Ser Lys Lys Glu Lys Val Ile
           115                 120                 125
   cta aag gaa cag aaa ctt cca gaa ata caa act cca ggt gta att gtg    432
   Leu Lys Glu Gln Lys Leu Pro Glu Ile Gln Thr Pro Gly Val Ile Val
       130                 135                 140
   aag gat gag aac atg cat act aan aag aaa ctt cca gaa ctc aca caa    480
   Lys Asp Glu Asn Met His Thr Xaa Lys Lys Leu Pro Glu Leu Thr Gln
   145 150 155 160
   gaa caa att att gac ata gtg aaa ctt ggt tta ctg gtt tgc tca gat 528
   Glu Gln Ile Ile Asp Ile Val Lys Leu Gly Leu Leu Val Cys Ser Asp
   165 170 175
   aaa aca cag gtt gcc acg tgt cca aat gat act aat tgt gga ata aac 576
   Lys Thr Gln Val Ala Thr Cys Pro Asn Asp Thr Asn Cys Gly Ile Asn
   180 185 190
   aat aat gta gta aac atg cct aag ctg caa act gtg gct taatttggga 625
   Asn Asn Val Val Asn Met Pro Lys Leu Gln Thr Val Ala
   195 200 205
   ttaatctcag attaattgtc tttccttttg agttaatttc ctagttctag ctttatcttg 685
   taatgt 691
<210> 8
   <211> 205
   <212> PRT
   <213> Solanum tuberosum
<400> 8
   Tyr Ala Pro Lys Lys Ala Arg Val Gln Thr Val Lys Met Leu Met Leu
   1 5 10 15
   Ser Val Val Gly Gly Phe Gly Ala Ile Ile Leu Val Thr Glu Leu Leu
   20 25 30
   Phe Lys Gly Ala Ile Arg Gly Gln Val Val Gly Trp Ile Cys Leu Ile
   35 40 45
   Phe Ser Leu Cys Val Phe Val Ala Pro Leu Gly Ile Val Arg Lys Val
   50 55 60
   Ile Lys Thr Lys Ser Val Glu Tyr Met Pro Leu Leu Leu Ser Val Phe
   65 70 75 80
   Leu Thr Leu Ser Ala Val Met Trp Phe Phe Tyr Gly Leu Leu Leu Lys
   85 90 95
   Asp Ile Asn Ile Ala Ala Pro Asn Val Leu Gly Phe Ile Phe Gly Ile
               100                 105                 110
   Leu Gln Ile Val Leu Tyr Ala Ile Tyr Ser Lys Lys Glu Lys Val Ile
           115                 120                 125
   Leu Lys Glu Gln Lys Leu Pro Glu Ile Gln Thr Pro Gly Val Ile Val
       130                 135                 140
   Lys Asp Glu Asn Met His Thr Xaa Lys Lys Leu Pro Glu Leu Thr Gln
   145                 150                 155                 160
   Glu Gln Ile Ile Asp Ile Val Lys Leu Gly Leu Leu Val Cys Ser Asp
                   165                 170                 175
   Lys Thr Gln Val Ala Thr Cys Pro Asn Asp Thr Asn Cys Gly Ile Asn
               180                 185                 190
   Asn Asn Val Val Asn Met Pro Lys Leu Gln Thr Val Ala
           195                 200                 205
<210> 9
   <211> 486
   <212> DNA
   <213> Solanum tuberosum
<220>
   <221> CDS
   <222> (3) .. (356)
<400> 9
   cc cct gga gtt gtt gta aat gat aat gga aga gga tac cgc aac ctg     47
      Pro Gly Val Val Val Asn Asp Asn Gly Arg Gly Tyr Arg Asn Leu
        1               5                  10                  15
   ttt gat gca ttc tta gat gct aca tac tca gcc ctt gaa aaa gcc ggt    95
   Phe Asp Ala Phe Leu Asp Ala Thr Tyr Ser Ala Leu Glu Lys Ala Gly
                    20                  25                  30
   ggc tca tct ttg gat atc gtt gta tca gag agc ggt tgg cct tca gct    143
   Gly Ser Ser Leu Asp Ile Val Val Ser Glu Ser Gly Trp Pro Ser Ala
               35                  40                  45
   gga gca ggg caa ttg acg tcc att gac aac gca aag act tac aac aac    191
   Gly Ala Gly Gln Leu Thr Ser Ile Asp Asn Ala Lys Thr Tyr Asn Asn
             50                  55                  60
   aac ttg att aga cat gtg aaa gga ggg agt ccg aaa aga cca tca aag    239
   Asn Leu Ile Arg His Val Lys Gly Gly Ser Pro Lys Arg Pro Ser Lys
       65                  70                  75
   cca att gaa gca tac att ttc gcg ctg ttg aat gaa gat ctg aag agc    287
   Pro Ile Glu Ala Tyr Ile Phe Ala Leu Leu Asn Glu Asp Leu Lys Ser
    80                  85                  90                  95
   cct gaa att gag aaa cat ttt gga ctg ttt aca gca agc agg cag cca    335
   Pro Glu Ile Glu Lys His Phe Gly Leu Phe Thr Ala Ser Arg Gln Pro
                   100                 105                 110
   aaa tac gcg atc agt ttc aac tagctagact atgctagata attaacggaa       386
   Lys Tyr Ala Ile Ser Phe Asn
               115
   taagagttgt cgatcttctc tctttataat atgtagttga ataatcgaat cgttgctcaa 446
   cttgaacgga gagcaaccct tcgcgtgata actgatgtgt                        486
<210> 10
   <211> 118
   <212> PRT
   <213> Solanum tuberosum
<400> 10
   Pro Gly Val Val Val Asn Asp Asn Gly Arg Gly Tyr Arg Asn Leu Phe
     1               5                  10                  15
   Asp Ala Phe Leu Asp Ala Thr Tyr Ser Ala Leu Glu Lys Ala Gly Gly
               20                  25                  30
   Ser Ser Leu Asp Ile Val Val Ser Glu Ser Gly Trp Pro Ser Ala Gly
           35                  40                  45
   Ala Gly Gln Leu Thr Ser Ile Asp Asn Ala Lys Thr Tyr Asn Asn Asn
       50                  55                  60
   Leu Ile Arg His Val Lys Gly Gly Ser Pro Lys Arg Pro Ser Lys Pro
    65                  70                  75                  80
   Ile Glu Ala Tyr Ile Phe Ala Leu Leu Asn Glu Asp Leu Lys Ser Pro
                   85                  90                  95
   Glu Ile Glu Lys His Phe Gly Leu Phe Thr Ala Ser Arg Gln Pro Lys
               100                 105                 110
   Tyr Ala Ile Ser Phe Asn
           115
<210> 11
   <211> 462
   <212> DNA
   <213> Solanum tuberosum
<220>
   <221> CDS
   <222> (1) .. (333)
<400> 11
   gaa act tta agg aga ttt gga tgg att gtt cca gca tac act atg cca     48
   Glu Thr Leu Arg Arg Phe Gly Trp Ile Val Pro Ala Tyr Thr Met Pro
     1               5                  10                  15
   cca gat gct caa cac acc aca gtt ctt aga gtt gtc atc aga gaa gat     96
   Pro Asp Ala Gln His Thr Thr Val Leu Arg Val Val Ile Arg Glu Asp
                20                  25                  30
   ttc tct aga aca ctc gct gag cga ttg gtt atg gac ata gtt aaa gtt     144
   Phe Ser Arg Thr Leu Ala Glu Arg Leu Val Met Asp Ile Val Lys Val
            35                  40                  45
   ctc cac gag ctc gac atg ctt cca gca cgt gtc aaa gct aag ctc gcc     192
   Leu His Glu Leu Asp Met Leu Pro Ala Arg Val Lys Ala Lys Leu Ala
        50                  55                  60
   atg gcc gag gag gcc tcg gcc gcg gcg aac ggt agc gac aat gca tcg     240
   Met Ala Glu Glu Ala Ser Ala Ala Ala Asn Gly Ser Asp Asn Ala Ser
    65                  70                  75                  80
   cat cat aaa acg gat aga gaa tgg gag tta gag gtt act gaa gca tgg     288
   His His Lys Thr Asp Arg Glu Trp Glu Leu Glu Val Thr Glu Ala Trp
                   85                  90                  95
   aag aag ttt gtt gct aag aaa aag aac aag act aag gga gtt tgt       333
   Lys Lys Phe Val Ala Lys Lys Lys Asn Lys Thr Lys Gly Val Cys
               100                 105                 110
   taattaattc ataaatattg tttatgagat gatgaattaa ttatgagttt atgatttaag 393
   tatttaataa tgagctcttt taatcgacaa tgtaaaattg gaccattatt aaagacaatt 453
   ttcttgtgt                                                          462
<210> 12
   <211> 111
   <212> PRT
   <213> Solanum tuberosum
<400> 12
   Glu Thr Leu Arg Arg Phe Gly Trp Ile Val Pro Ala Tyr Thr Met Pro
     1               5                  10                  15
   Pro Asp Ala Gln His Thr Thr Val Leu Arg Val Val Ile Arg Glu Asp
               20                  25                  30
   Phe Ser Arg Thr Leu Ala Glu Arg Leu Val Met Asp Ile Val Lys Val
           35                  40                  45
   Leu His Glu Leu Asp Met Leu Pro Ala Arg Val Lys Ala Lys Leu Ala
       50                  55                  60
   Met Ala Glu Glu Ala Ser Ala Ala Ala Asn Gly Ser Asp Asn Ala Ser
   65                  70                  75                  80
   His His Lys Thr Asp Arg Glu Trp Glu Leu Glu Val Thr Glu Ala Trp
                   85                  90                  95
   Lys Lys Phe Val Ala Lys Lys Lys Asn Lys Thr Lys Gly Val Cys
               100                 105                 110
<210> 13
   <211> 394
   <212> DNA
   <213> Solanum tuberosum
<220>
   <221> CDS
   <222> (3) .. (323)
<400> 13
   ac aat ccg cag aac ata aac tgg gat ttg aga act gct agc gct tac     47
      Asn Pro Gln Asn Ile Asn Trp Asp Leu Arg Thr Ala Ser Ala Tyr
        1               5                   10                  15
   tgc gct act tgg gat gct gac aag cct cta gcg tgg cgc cag agg tat    95
   Cys Ala Thr Trp Asp Ala Asp Lys Pro Leu Ala Trp Arg Gln Arg Tyr
                       20                  25                  30
   ggc tgg acc gct ttt tgt ggt cca gct gga cct agg ggc caa gct tca    143
   Gly Trp Thr Ala Phe Cys Gly Pro Ala Gly Pro Arg Gly Gln Ala Ser
                   35                  40                  45
   tgc ggt aga tgc ttg agg gtg aca aac aca gga aca gga aca caa gaa    191
   Cys Gly Arg Cys Leu Arg Val Thr Asn Thr Gly Thr Gly Thr Gln Glu
               50                  55                  60
   aca gtg aga ata gta gat caa tgc agc aac gga ggg ctt gat ttg gat    239
   Thr Val Arg Ile Val Asp Gln Cys Ser Asn Gly Gly Leu Asp Leu Asp
           65                  70                  75
   gta aac gtg ttt aac cga ttg gac act aat gga ttg ggc tac cag agg    287
   Val Asn Val Phe Asn Arg Leu Asp Thr Asn Gly Leu Gly Tyr Gln Arg
    80                  85                  90                  95
   ggc aac ctt aat gtt aac tat gaa ttt gtc aac tgc taagcttaaa         333
   Gly Asn Leu Asn Val Asn Tyr Glu Phe Val Asn Cys
                   100                 105
   aaagtgtcat atatcatcaa taaaataata aaccacgatc gagattgatt tcgtcctatg 393
   t                                                                  394
<210> 14
   <211> 107
   <212> PRT
<213> Solanum tuberosum
<400> 14
   Asn Pro Gln Asn Ile Asn Trp Asp Leu Arg Thr Ala Ser Ala Tyr Cys
   1 5 10 15
   Ala Thr Trp Asp Ala Asp Lys Pro Leu Ala Trp Arg Gln Arg Tyr Gly
   20 25 30
   Trp Thr Ala Phe Cys Gly Pro Ala Gly Pro Arg Gly Gln Ala Ser Cys
   35 40 45
   Gly Arg Cys Leu Arg Val Thr Asn Thr Gly Thr Gly Thr Gln Glu Thr
   50 55 60
   Val Arg Ile Val Asp Gln Cys Ser Asn Gly Gly Leu Asp Leu Asp Val
   65 70 75 80
   Asn Val Phe Asn Arg Leu Asp Thr Asn Gly Leu Gly Tyr Gln Arg Gly
   85 90 95
   Asn Leu Asn Val Asn Tyr Glu Phe Val Asn Cys
   100 105
<210> 15
   <211> 447
   <212> DNA
   <213> Solanum tuberosum
<220>
   <221> CDS
   <222> (3) .. (407)
<400> 15
   cc caa aat tca ccc caa gac tat ctt gca gtt cac aac gat gcc cgt 47
   Gln Asn Ser Pro Gln Asp Tyr Leu Ala Val His Asn Asp Ala Arg
   1 5 10 15
   gcc caa gtn ggg gtt ggg cca atg tca tgg gat gcc agc ttg gca acc 95
   Ala Gln Val Gly Val Gly Pro Met Ser Trp Asp Ala Ser Leu Ala Thr
   20 25 30
   caa gca caa aac tat gcc aac tca aga att ggt gat tgc aac ttg att 143
   Gln Ala Gln Asn Tyr Ala Asn Ser Arg Ile Gly Asp Cys Asn Leu Ile
   35 40 45
   cat tct ggt gct ggg gag aac ctt gcc aag ggt gga gga gac tac aca 191
   His Ser Gly Ala Gly Glu Asn Leu Ala Lys Gly Gly Gly Asp Tyr Thr
   50 55 60
   ggg agg cgt gct gtg caa ttg tgg gtg tca gaa aag cca aac tac aac 239
   Gly Arg Arg Ala Val Gln Leu Trp Val Ser Glu Lys Pro Asn Tyr Asn
   65 70 75
   tat gct acc aac caa tgt gct agt gga aaa gtg tgc gga cac tat act 287
   Tyr Ala Thr Asn Gln Cys Ala Ser Gly Lys Val Cys Gly His Tyr Thr
   80 85 90 95
   caa gta gtt tgg cgc aac tca gtt cga cta ggt tgt ggt cgg gct cgt 335
   Gln Val Val Trp Arg Asn Ser Val Arg Leu Gly Cys Gly Arg Ala Arg
   100 105 110
   tgc aac aat ggt tgg tgg ttc att tct tgc aac tac gat cct gta ggc 383
   Cys Asn Asn Gly Trp Trp Phe Ile Ser Cys Asn Tyr Asp Pro Val Gly
   115 120 125
   aat tgg gtc gga caa cgt cct tac taaaaatgtg tttacttatg atgtgtaagg 437
   Asn Trp Val Gly Gln Arg Pro Tyr
   130 135
   gattaaatgt 447
<210> 16
   <211> 135
   <212> PRT
   <213> Solanum tuberosum
<400> 16
   Gln Asn Ser Pro Gln Asp Tyr Leu Ala Val His Asn Asp Ala Arg Ala
   1 5 10 15
   Gln Val Gly Val Gly Pro Met Ser Trp Asp Ala Ser Leu Ala Thr Gln
   20 25 30
   Ala Gln Asn Tyr Ala Asn Ser Arg Ile Gly Asp Cys Asn Leu Ile His
   35 40 45
   Ser Gly Ala Gly Glu Asn Leu Ala Lys Gly Gly Gly Asp Tyr Thr Gly
       50                  55                  60
   Arg Arg Ala Val Gln Leu Trp Val Ser Glu Lys Pro Asn Tyr Asn Tyr
   65                  70                  75                  80
   Ala Thr Asn Gln Cys Ala Ser Gly Lys Val Cys Gly His Tyr Thr Gln
                   85                  90                  95
   Val Val Trp Arg Asn Ser Val Arg Leu Gly Cys Gly Arg Ala Arg Cys
               100                 105                 110
   Asn Asn Gly Trp Trp Phe Ile Ser Cys Asn Tyr Asp Pro Val Gly Asn
           115                 120                 125
   Trp Val Gly Gln Arg Pro Tyr
       130                 135
<210> 17
   <211> 501
   <212> DNA
   <213> Solanum tuberosum
<220>
   <221> CDS
   <222> (3) .. (500)
<400> 17
   ct agg gaa tta ttc gaa caa atg ttg agt ttt agg aac aat gat gta     47
      Arg Glu Leu Phe Glu Gln Met Leu Ser Phe Arg Asn Asn Asp Val
        1               5                  10                  15
   tgt cct ggc aaa gga ttc tac act tac gat gca ttc ata gct gca gct    95
   Cys Pro Gly Lys Gly Phe Tyr Thr Tyr Asp Ala Phe Ile Ala Ala Ala
                   20                  25                  30
   aac tcc ttt cca gct ttt ggt gct act ggt gat gat acc gcc cgt aag    143
   Asn Ser Phe Pro Ala Phe Gly Ala Thr Gly Asp Asp Thr Ala Arg Lys
               35                  40                  45
   aag gaa atg gct gcc ttt ttc ggt caa aca tct cac gaa act aaa ggt    191
   Lys Glu Met Ala Ala Phe Phe Gly Gln Thr Ser His Glu Thr Lys Gly
           50                  55                  60
   ggt agt gca gga aca ttt act gga gga tat tgc ttt gtt agg caa ata    239
   Gly Ser Ala Gly Thr Phe Thr Gly Gly Tyr Cys Phe Val Arg Gln Ile
       65                  70                  75
   gat cag tca gaa aga tac tat ggc aga gga cct att caa ttg aca cac    287
   Asp Gln Ser Glu Arg Tyr Tyr Gly Arg Gly Pro Ile Gln Leu Thr His
    80                  85                  90                  95
   caa tct aac tac gaa cga gct aga cag ggt att ggt gtt gga caa gac    335
   Gln Ser Asn Tyr Glu Arg Ala Arg Gln Gly Ile Gly Val Gly Gln Asp
                   100                 105                 110
   cta gtt aat aac cct gat tta gta gcc aca gat cct ata ata tca ttc    383
   Leu Val Asn Asn Pro Asp Leu Val Ala Thr Asp Pro Ile Ile Ser Phe
               115                 120                 125
   aaa aca gca att tgg ttc tgg atg aca gca cag gat aat aag cca tca    431
   Lys Thr Ala Ile Trp Phe Trp Met Thr Ala Gln Asp Asn Lys Pro Ser
           130                 135                 140
   tgt cac agc gtt atc att ggg caa tgg acg cca tcc cct gca gat acg    479
   Cys His Ser Val Ile Ile Gly Gln Trp Thr Pro Ser Pro Ala Asp Thr
       145                 150                 155
   gca gct aat aga gtg cca ggg t                                      501
   Ala Ala Asn Arg Val Pro Gly
   160                 165
<210> 18
   <211> 166
   <212> PRT
   <213> Solanum tuberosum
<400> 18
   Arg Glu Leu Phe Glu Gln Met Leu Ser Phe Arg Asn Asn Asp Val Cys
    1               5                  10                  15
   Pro Gly Lys Gly Phe Tyr Thr Tyr Asp Ala Phe Ile Ala Ala Ala Asn
               20                  25                  30
   Ser Phe Pro Ala Phe Gly Ala Thr Gly Asp Asp Thr Ala Arg Lys Lys
           35                  40                  45
   Glu Met Ala Ala Phe Phe Gly Gln Thr Ser His Glu Thr Lys Gly Gly
       50                  55                  60
   Ser Ala Gly Thr Phe Thr Gly Gly Tyr Cys Phe Val Arg Gln Ile Asp
   65                  70                  75                  80
   Gln Ser Glu Arg Tyr Tyr Gly Arg Gly Pro Ile Gln Leu Thr His Gln
                   85                  90                  95
   Ser Asn Tyr Glu Arg Ala Arg Gln Gly Ile Gly Val Gly Gln Asp Leu
               100                 105                 110
   Val Asn Asn Pro Asp Leu Val Ala Thr Asp Pro Ile Ile Ser Phe Lys
           115                 120                 125
   Thr Ala Ile Trp Phe Trp Met Thr Ala Gln Asp Asn Lys Pro Ser Cys
       130                 135                 140
   His Ser Val Ile Ile Gly Gln Trp Thr Pro Ser Pro Ala Asp Thr Ala
   145                 150                 155                 160
   Ala Asn Arg Val Pro Gly
                   165
<210> 19
   <211> 337
   <212> DNA
   <213> Solanum tuberosum
<220>
   <221> CDS
   <222> (1) .. (336)
<400> 19
   aca gct gaa aca ttg acc atg gat tat cac aga ggt gct gaa gtt gag    48
   Thr Ala Glu Thr Leu Thr Met Asp Tyr His Arg Gly Ala Glu Val Glu
   1               5                   10                  15
   gtg aga att gct agg att ttt aac act tat gga cct cgt atg tgt att    96
   Val Arg Ile Ala Arg Ile Phe Asn Thr Tyr Gly Pro Arg Met Cys Ile
               20                  25                  30
   gat gat ggt cgc gtt gtc agc aac ttt gtc gct cag gct tta agg aaa    144
   Asp Asp Gly Arg Val Val Ser Asn Phe Val Ala Gln Ala Leu Arg Lys
               35                  40                  45
   gag cct ttg aca gtt tat ggt gat ggg aaa caa aca aga agt ttc caa    192
   Glu Pro Leu Thr Val Tyr Gly Asp Gly Lys Gln Thr Arg Ser Phe Gln
       50                  55                  60
   ttt gtt tca gat ctg gtt gaa ggc cta atg cgg ttg atg gaa gga gaa    240
   Phe Val Ser Asp Leu Val Glu Gly Leu Met Arg Leu Met Glu Gly Glu
    65                  70                  75                  80
   cat gtc gga ccc ttc aac ctt gga aac cct ggt gaa ttc caa tgc ttg    288
   His Val Gly Pro Phe Asn Leu Gly Asn Pro Gly Glu Phe Gln Cys Leu
                   85                  90                  95
   aac tcg cta agg tng tcc agg aaa cca ttg acc caa atg cac aga ttg t 337
   Asn Ser Leu Arg Xaa Ser Arg Lys Pro Leu Thr Gln Met His Arg Leu
               100                 105                 110
<210> 20
   <211> 112
   <212> PRT
   <213> Solanum tuberosum
<400> 20
   Thr Ala Glu Thr Leu Thr Met Asp Tyr His Arg Gly Ala Glu Val Glu
    1               5                  10                  15
   Val Arg Ile Ala Arg Ile Phe Asn Thr Tyr Gly Pro Arg Met Cys Ile
               20                  25                  30
   Asp Asp Gly Arg Val Val Ser Asn Phe Val Ala Gln Ala Leu Arg Lys
           35                  40                  45
   Glu Pro Leu Thr Val Tyr Gly Asp Gly Lys Gln Thr Arg Ser Phe Gln
       50                  55                  60
   Phe Val Ser Asp Leu Val Glu Gly Leu Met Arg Leu Met Glu Gly Glu
    65                  70                  75                  80
   His Val Gly Pro Phe Asn Leu Gly Asn Pro Gly Glu Phe Gln Cys Leu
                   85                  90                  95
   Asn Ser Leu Arg Xaa Ser Arg Lys Pro Leu Thr Gln Met His Arg Leu
               100                 105                 110
<210> 21
   <211> 565
   <212> DNA
   <213> Solanum tuberosum
<220>
   <221> CDS
   <222> (3) .. (299)
<400> 21
   ga caa ttt gag gga tat ttt tat gtg cca agt ggt tca tct ggt gtg    47
      Gln Phe Glu Gly Tyr Phe Tyr Val Pro Ser Gly Ser Ser Gly Val
      1               5                   10                  15
   tca atc atg caa gta ttt gga gca tca cca tca tcc aca act tta atg   95
   Ser Ile Met Gln Val Phe Gly Ala Ser Pro Ser Ser Thr Thr Leu Met
                       20                  25                  30
   tta aga gtt tat gat gga act ctc tat tac tat aga gaa aaa gtt att   143
   Leu Arg Val Tyr Asp Gly Thr Leu Tyr Tyr Tyr Arg Glu Lys Val Ile
               35                  40                  45
   gaa tca aat att tat aat aga tgg atc aga cta aat gtg att cat gat   191
   Glu Ser Asn Ile Tyr Asn Arg Trp Ile Arg Leu Asn Val Ile His Asp
           50                  55                  60
   gtt ggt gcg agt aaa tta aag gtt tat att aat gga aat ctc aag cat   239
   Val Gly Ala Ser Lys Leu Lys Val Tyr Ile Asn Gly Asn Leu Lys His
       65                  70                  75
   gaa cca tcc gga aga ngt gga gac cat cat tat ttc aaa ttt ggg agt   287
   Glu Pro Ser Gly Arg Xaa Gly Asp His His Tyr Phe Lys Phe Gly Ser
   80                  85                  90                  95
   tta tac aca aga taatgagtct gatcgaatgg agtctcgttg gagagggatt       339
   Leu Tyr Thr Arg
   aagcttttca agaaataaac atctatcact tgtagtgtct taacatgatc gagcggacta 399
   gctctatatg aatcatgaaa cttatgttat ttcatttaat taagaaatat gatgttttta 459
   gacnaatnaa tctcctctta gatttatgat cttgaagttg aattgaataa gatttatnaa 519
   aagaaatgaa acnagcttta atccaaaaaa aaaaaaaaaa aaaaaa                565
<210> 22
   <211> 99
   <212> PRT
   <213> Solanum tuberosum
<400> 22
   Gln Phe Glu Gly Tyr Phe Tyr Val Pro Ser Gly Ser Ser Gly Val Ser
    1               5                  10                  15
   Ile Met Gln Val Phe Gly Ala Ser Pro Ser Ser Thr Thr Leu Met Leu
               20                  25                  30
   Arg Val Tyr Asp Gly Thr Leu Tyr Tyr Tyr Arg Glu Lys Val Ile Glu
           35                  40                  45
   Ser Asn Ile Tyr Asn Arg Trp Ile Arg Leu Asn Val Ile His Asp Val
       50                  55                  60
   Gly Ala Ser Lys Leu Lys Val Tyr Ile Asn Gly Asn Leu Lys His Glu
   65                  70                  75                  80
   Pro Ser Gly Arg Xaa Gly Asp His His Tyr Phe Lys Phe Gly Ser Leu
                   85                  90                  95
   Tyr Thr Arg
<210> 23
   <211> 291
   <212> DNA
   <213> Solanum tuberosum
<220>
   <221> CDS
   <222> (3)..(212)
<400> 23
   ac ata cca ttt ggt gca gga aga agg agg tgt cct ggc atg tca ttt     47
   Ile Pro Phe Gly Ala Gly Arg Arg Arg Cys Pro Gly Met Ser Phe
    1               5                  10                  15
   ggt cta gca aat gta gag cat cca ttg gct ctt ttt cta tac cat ttt    95
   Gly Leu Ala Asn Val Glu His Pro Leu Ala Leu Phe Leu Tyr His Phe
                   20                  25                  30
   gat tgg aag cta ccc aat ggg atg aaa cat gaa gaa ttg gat atg agg    143
   Asp Trp Lys Leu Pro Asn Gly Met Lys His Glu Glu Leu Asp Met Arg
               35                  40                  45
   gag gtg aat ctt ggt gtg tca gtg aaa aga gaa ctt gat atg cat gta    191
   Glu Val Asn Leu Gly Val Ser Val Lys Arg Glu Leu Asp Met His Val
           50                  55                  60
   att cct att gtg acc aat cca gtgtaacaat aataaaagca tctatataac       242
   Ile Pro Ile Val Thr Asn Pro
       65                  70
   ttcgcacttt ctaataaaaa aaaaaaaaaa aaaaaaaaaa aaaaaaaaa              291
<210> 24
   <211> 70
   <212> PRT
   <213> Solanum tuberosum
<400> 24
   Ile Pro Phe Gly Ala Gly Arg Arg Arg Cys Pro Gly Met Ser Phe Gly
    1               5                  10                  15
   Leu Ala Asn Val Glu His Pro Leu Ala Leu Phe Leu Tyr His Phe Asp
               20                  25                  30
   Trp Lys Leu Pro Asn Gly Met Lys His Glu Glu Leu Asp Met Arg Glu
           35                  40                  45
   Val Asn Leu Gly Val Ser Val Lys Arg Glu Leu Asp Met His Val Ile
       50                  55                  60
   Pro Ile Val Thr Asn Pro
   65                  70
<210> 25
   <211> 554
   <212> DNA
   <213> Solanum tuberosum
<220>
   <221> CDS
   <222> (1) .. (474)
<400> 25
   att aag ctc aaa cta tat gat cct tca gaa ttc cat gtg att aat tct    48
   Ile Lys Leu Lys Leu Tyr Asp Pro Ser Glu Phe His Val Ile Asn Ser
    1               5                  10                  15
   aat aag aaa tca aga gtt ggg aac cct gtt ggt tac aaa gtt gtt cct    96
   Asn Lys Lys Ser Arg Val Gly Asn Pro Val Gly Tyr Lys Val Val Pro
               20                  25                  30
   ggt gga act gct gct agt tta ctt gac cat gat gat tct cca cag aag    144
   Gly Gly Thr Ala Ala Ser Leu Leu Asp His Asp Asp Ser Pro Gln Lys
           35                  40                  45
   aga gct gct ttt act aat aac caa att tgg gtc aca cct tat aat gaa    192
   Arg Ala Ala Phe Thr Asn Asn Gln Ile Trp Val Thr Pro Tyr Asn Glu
       50                  55                  60
   tct gag caa tgg gct gct ggt ttg ttt gtt tat caa agt caa ggt gat    240
   Ser Glu Gln Trp Ala Ala Gly Leu Phe Val Tyr Gln Ser Gln Gly Asp
    65                  70                  75                  80
   nac act ctt gct gtt tgg tct gac aga gac agg gca att gag aat aaa    288
   Xaa Thr Leu Ala Val Trp Ser Asp Arg Asp Arg Ala Ile Glu Asn Lys
                   85                  90                  95
   gac ata gtg gtg tgg tat aca tta ggg ttc cat cat att cca tgc caa    336
   Asp Ile Val Val Trp Tyr Thr Leu Gly Phe His His Ile Pro Cys Gln
               100                 105                 110
   gag gat ttc cca ata atg cca aca gta tca tca agt ttt gag ata aag    384
   Glu Asp Phe Pro Ile Met Pro Thr Val Ser Ser Ser Phe Glu Ile Lys
           115                 120                 125
   cca gtt aat ttc ttt gag agt aac cca att ctc aat att cca cct aat   432
   Pro Val Asn Phe Phe Glu Ser Asn Pro Ile Leu Asn Ile Pro Pro Asn
       130                 135                 140
   tct cca aag gac ctc cca att tgc aaa gct gct gct tct gct           474
   Ser Pro Lys Asp Leu Pro Ile Cys Lys Ala Ala Ala Ser Ala
   145                 150                 155
   tgatcatact ttaattgtat tgtatgtgca aactntgtaa taataatnac nattgccgcg 534
   ctagacttgg gatttcgggt                                              554
<210> 26
   <211> 158
   <212> PRT
   <213> Solanum tuberosum
<400> 26
   Ile Lys Leu Lys Leu Tyr Asp Pro Ser Glu Phe His Val Ile Asn Ser
   1               5                   10                  15
   Asn Lys Lys Ser Arg Val Gly Asn Pro Val Gly Tyr Lys Val Val Pro
               20                  25                  30
   Gly Gly Thr Ala Ala Ser Leu Leu Asp His Asp Asp Ser Pro Gln Lys
           35                  40                  45
   Arg Ala Ala Phe Thr Asn Asn Gln Ile Trp Val Thr Pro Tyr Asn Glu
       50                  55                  60
   Ser Glu Gln Trp Ala Ala Gly Leu Phe Val Tyr Gln Ser Gln Gly Asp
   65                  70                  75                  80
   Xaa Thr Leu Ala Val Trp Ser Asp Arg Asp Arg Ala Ile Glu Asn Lys
                   85                  90                  95
   Asp Ile Val Val Trp Tyr Thr Leu Gly Phe His His Ile Pro Cys Gln
               100                 105                 110
   Glu Asp Phe Pro Ile Met Pro Thr Val Ser Ser Ser Phe Glu Ile Lys
           115                 120                 125
   Pro Val Asn Phe Phe Glu Ser Asn Pro Ile Leu Asn Ile Pro Pro Asn
       130                 135                 140
   Ser Pro Lys Asp Leu Pro Ile Cys Lys Ala Ala Ala Ser Ala
   145                 150                 155
<210> 27
   <211> 371
   <212> DNA
   <213> Solanum tuberosum
<220>
   <221> CDS
   <222> (4) .. (96)
<400> 27
   ana atg agc tac caa agg cct atc tta ctg aaa tgc tca cat gta ata   48
       Met Ser Tyr Gln Arg Pro Ile Leu Leu Lys Cys Ser His Val Ile
         1               5                  10                  15
   cta cta act ttc tct ggt tgt gat gtt ttt cta gtc cat ttc agt cta   96
   Leu Leu Thr Phe Ser Gly Cys Asp Val Phe Leu Val His Phe Ser Leu
                       20                  25                  30
   taatgatgtg tgtgagatga aagcttcttg gcnatttctg ttataaacct actctgtagt 156
   ctctgtgtcc cattttacgt ggaagttatc gttccagcgt tggatcttta cttattatgg 216
   agcaggttag caccatatgt gttttcattt tcatgtaatg aagaagctaa acctgtattc 276
   tttctcttcc ttgttgagct agctgaaaac attgatcaaa gatatgcagt caagattgag 336
   ggtgtttttc cnaaaaaaaa aaaaaaaaaa aaaaa                            371
<210> 28
   <211> 31
   <212> PRT
   <213> Solanum tuberosum
<400> 28
   Met Ser Tyr Gln Arg Pro Ile Leu Leu Lys Cys Ser His Val Ile Leu
     1               5                  10                  15
   Leu Thr Phe Ser Gly Cys Asp Val Phe Leu Val His Phe Ser Leu
               20                  25                  30
<210> 29
   <211> 296
   <212> DNA
   <213> Solanum tuberosum
<400> 29
   acaatgaatt caatggtaag aacataaacg ttgaacattt gaatcctaaa tctgcctaac 60
   acatatgtaa tataggtcca acgtattcaa ctgaactcaa catttttcct atgcggaaat 120
   caaacataat atgtgagaaa ttactatatt tccactaaca attcattttg aactcataat 180
   tccaaaagta taactattga actccaacat tcaaatcctg aattcgtctc tactcctgga 240
   gacgtgattc ttgatcctat aattgttttt tgtttttcaa ttttgtctag gtttgt 296
<210> 30
   <211> 432
   <212> DNA
   <213> Solanum tuberosum
<220>
   <221> CDS
   <222> (1)..(261)
<400> 30
   aaa aaa atg gnt tcc aag gca att atg ttt ctt gtt ctt ttt tgg gct 48
   Lys Lys Met Xaa Ser Lys Ala Ile Met Phe Leu Val Leu Phe Trp Ala
   1 5 10 15
   att ttc tta atg ata agn tct gag gtt gct gct agg gag ttg gca gct 96
   Ile Phe Leu Met Ile Xaa Ser Glu Val Ala Ala Arg Glu Leu Ala Ala
   20 25 30
   gag act tcc aat gcg gta aac gtt gat gga cat tat tat ggt ggt ggc 144
   Glu Thr Ser Asn Ala Val Asn Val Asp Gly His Tyr Tyr Gly Gly Gly
   35 40 45
   tat cac aag ggc tat gga aaa cct aag aaa tgt tat agt tgc tgc aaa 192
   Tyr His Lys Gly Tyr Gly Lys Pro Lys Lys Cys Tyr Ser Cys Cys Lys
        50                  55                  60
   aaa tac aaa tat gga tgc aaa aaa tac tgc tgc tct tat gaa gaa tat    240
   Lys Tyr Lys Tyr Gly Cys Lys Lys Tyr Cys Cys Ser Tyr Glu Glu Tyr
    65                 70                  75                  80
   gtg gct gcc cag act cac aac taaataatta attagtgctt gattattatg       291
   Val Ala Ala Gln Thr His Asn
                       85
   tgtattatta ttattgtaaa cttttgaaat taagtgacaa gataataata atcttgttac 351
   ttaagaccct ttgctgtaac aagtatgaat aaagccattc atttactatg aatggttggt 411
   catgatgtaa tgttttgttg t                                            432
<210> 31
   <211> 87
   <212> PRT
   <213> Solanum tuberosum
<400> 31
   Lys Lys Met Xaa Ser Lys Ala Ile Met Phe Leu Val Leu Phe Trp Ala
    1               5                  10                  15
   Ile Phe Leu Met Ile Xaa Ser Glu Val Ala Ala Arg Glu Leu Ala Ala
               20                  25                  30
   Glu Thr Ser Asn Ala Val Asn Val Asp Gly His Tyr Tyr Gly Gly Gly
           35                  40                  45
   Tyr His Lys Gly Tyr Gly Lys Pro Lys Lys Cys Tyr Ser Cys Cys Lys
       50                  55                  60
   Lys Tyr Lys Tyr Gly Cys Lys Lys Tyr Cys Cys Ser Tyr Glu Glu Tyr
    65                 70                  75                  80
   Val Ala Ala Gln Thr His Asn
                       85
<210> 32
   <211> 297
   <212> DNA
   <213> Solanum tuberosum
<400> 32
   acctcctcgc taatccttct gtctacgctt aagtgatgaa atatgaatca gatccacact 60
   tgagagatga tcaggacgtg ttgcgatgct atataattaa ataattatat atacgtgtgt 120
   tctatttcta aaaagtttaa acttttagct gagacgaaaa agttgatcaa aagttccatg 180
   ggcttgagtt tttacactgt tatagttttt tgttgtgtgt tttgttcttt ccaattgtat 240
   gacatggaaa gaacttccaa taaataatat tgaaagcttt gctttctttt tgcttgt    297
<210> 33
   <211> 556
   <212> DNA
   <213> Solanum tuberosum
<220>
   <221> CDS
   <222> (59) .. (523)
<400> 33
   naaactattt aatgnaacaa ttatnttggg aggtcaatca attttcaagt tccactag   58
   gtg atg caa ttt tta gat gga att gat ttt gat ata gaa aga gga aca   106
   Val Met Gln Phe Leu Asp Gly Ile Asp Phe Asp Ile Glu Arg Gly Thr
     1               5                  10                  15
   aca caa cac tgg gat gaa cta gca aga gcc ctc tca gga ttt agc caa   154
   Thr Gln His Trp Asp Glu Leu Ala Arg Ala Leu Ser Gly Phe Ser Gln
                   20                  25                  30
   caa agg aaa gta tac tta act gca gct cca caa tgt cca ttc cct gat   202
   Gln Arg Lys Val Tyr Leu Thr Ala Ala Pro Gln Cys Pro Phe Pro Asp
               35                  40                  45
   tca tgg tta aat ggt gca ctc tct act gga cta ttn gac tac gtt tgg   250
   Ser Trp Leu Asn Gly Ala Leu Ser Thr Gly Leu Xaa Asp Tyr Val Trp
           50                  55                  60
   gtt caa ttc tac aac aat ccc ccc tgt caa tgc tct ggt ggt agc gcg   298
   Val Gln Phe Tyr Asn Asn Pro Pro Cys Gln Cys Ser Gly Gly Ser Ala
    65                  70                  75                  80
   gaa aat ctt aag agt tat tgg aat aaa tgg acc gcg att caa gct gga   346
   Glu Asn Leu Lys Ser Tyr Trp Asn Lys Trp Thr Ala Ile Gln Ala Gly
                   85                  90                  95
   aag att ttt cta gga ttg ccc gcg gct cca gga gct gct ggg agt ggt   394
   Lys Ile Phe Leu Gly Leu Pro Ala Ala Pro Gly Ala Ala Gly Ser Gly
               100                 105                 110
   ttt ata cca tct gat gtg ctt gtt tct cag gtt tta cca gca att agt   442
   Phe Ile Pro Ser Asp Val Leu Val Ser Gln Val Leu Pro Ala Ile Ser
           115                 120                 125
   ggt tca cct aag tat ggt ggt gtc atg ctt tgg tct aaa ttt ttt gat   490
   Gly Ser Pro Lys Tyr Gly Gly Val Met Leu Trp Ser Lys Phe Phe Asp
       130                 135                 140
   aat ggt tat agc tct gct ata aag cct agt gtc taagatgtat gtccatatag 543
   Asn Gly Tyr Ser Ser Ala Ile Lys Pro Ser Val
   145                 150                 155
   ttggttatgt tgt                                                    556
<210> 34
   <211> 155
   <212> PRT
   <213> Solanum tuberosum
<400> 34
   Val Met Gln Phe Leu Asp Gly Ile Asp Phe Asp Ile Glu Arg Gly Thr
     1               5                  10                  15
   Thr Gln His Trp Asp Glu Leu Ala Arg Ala Leu Ser Gly Phe Ser Gln
                20                  25                  30
   Gln Arg Lys Val Tyr Leu Thr Ala Ala Pro Gln Cys Pro Phe Pro Asp
            35                  40                  45
   Ser Trp Leu Asn Gly Ala Leu Ser Thr Gly Leu Xaa Asp Tyr Val Trp
           50                  55                  60
   Val Gln Phe Tyr Asn Asn Pro Pro Cys Gln Cys Ser Gly Gly Ser Ala
   65                  70                  75                  80
   Glu Asn Leu Lys Ser Tyr Trp Asn Lys Trp Thr Ala Ile Gln Ala Gly
                   85                  90                  95
   Lys Ile Phe Leu Gly Leu Pro Ala Ala Pro Gly Ala Ala Gly Ser Gly
               100                 105                 110
   Phe Ile Pro Ser Asp Val Leu Val Ser Gln Val Leu Pro Ala Ile Ser
           115                 120                 125
   Gly Ser Pro Lys Tyr Gly Gly Val Met Leu Trp Ser Lys Phe Phe Asp
       130                 135                 140
   Asn Gly Tyr Ser Ser Ala Ile Lys Pro Ser Val
   145                 150                 155
<210> 35
   <211> 472
   <212> DNA
   <213> Solanum tuberosum
<220>
   <221> CDS
   <222> (2) .. (370)
<400> 35
   g aat atg cta atc att ggc ata tct ata gtc atc aca agt agt gct gca 49
     Asn Met Leu Ile Ile Gly Ile Ser Ile Val Ile Thr Ser Ser Ala Ala
       1               5                   10                  15
   ttt gtt tgg aac aag aga cag agt ggc act gat gca aaa cag att cag   97
   Phe Val Trp Asn Lys Arg Gln Ser Gly Thr Asp Ala Lys Gln Ile Gln
                   20                  25                  30
   aac atg gaa ggt gca act cct atg gct tcg cct aat tct tgg ttc tat   145
   Asn Met Glu Gly Ala Thr Pro Met Ala Ser Pro Asn Ser Trp Phe Tyr
             35                  40                  45
   aat gct gat agg gag atg gaa agt ttt ccc caa cag tca ctt ttt caa   193
   Asn Ala Asp Arg Glu Met Glu Ser Phe Pro Gln Gln Ser Leu Phe Gln
       50                  55                  60
   tca act aat ctc cat ttt ggt gaa agg cct gac ctc aaa aga cta ttg    241
   Ser Thr Asn Leu His Phe Gly Glu Arg Pro Asp Leu Lys Arg Leu Leu
    65                  70                  75                  80
   ttt ggg agg aaa gaa tca agt gtt gga gtt tta gtg tgt ggc cca aaa    289
   Phe Gly Arg Lys Glu Ser Ser Val Gly Val Leu Val Cys Gly Pro Lys
                   85                  90                  95
   aag atg aga cat gaa gtt gct acc ata tgt tca tct ggt tta gca tcc    337
   Lys Met Arg His Glu Val Ala Thr Ile Cys Ser Ser Gly Leu Ala Ser
               100                 105                 110
   aac ctt cat ttt gag tct atc agt ttc agt tgg tgatttagag aatataatgc 390
   Asn Leu His Phe Glu Ser Ile Ser Phe Ser Trp
           115                 120
   aatggaaaaa taagatttgt ttgtgtgctt atgctttggt tgattgaatt ttgcaagtgc 450
   tcctgtaaca tgcttccttt gt                                           472
<210> 36
   <211> 123
   <212> PRT
   <213> Solanum tuberosum
<400> 36
   Asn Met Leu Ile Ile Gly Ile Ser Ile Val Ile Thr Ser Ser Ala Ala
     1               5                  10                  15
   Phe Val Trp Asn Lys Arg Gln Ser Gly Thr Asp Ala Lys Gln Ile Gln
               20                  25                  30
   Asn Met Glu Gly Ala Thr Pro Met Ala Ser Pro Asn Ser Trp Phe Tyr
           35                  40                  45
   Asn Ala Asp Arg Glu Met Glu Ser Phe Pro Gln Gln Ser Leu Phe Gln
       50                  55                  60
   Ser Thr Asn Leu His Phe Gly Glu Arg Pro Asp Leu Lys Arg Leu Leu
    65                  70                  75                  80
   Phe Gly Arg Lys Glu Ser Ser Val Gly Val Leu Val Cys Gly Pro Lys
                   85                  90                  95
   Lys Met Arg His Glu Val Ala Thr Ile Cys Ser Ser Gly Leu Ala Ser
               100                 105                 110
   Asn Leu His Phe Glu Ser Ile Ser Phe Ser Trp
           115                 120
<210> 37
   <211> 498
   <212> DNA
   <213> Solanum tuberosum
<220>
   <221> CDS
   <222> (2) .. (496)
<400> 37
   c ttc ctt tat ttt ggt caa tac tat ttn tat gtc nct cat nct gnc act 49
     Phe Leu Tyr Phe Gly Gln Tyr Tyr Xaa Tyr Val Xaa His Xaa Xaa Thr
     1               5                   10                  15
   ntt gac att acc aat cgg tgc acc tac ccg gtc tgg gcc gct gcc tct   97
   Xaa Asp Ile Thr Asn Arg Cys Thr Tyr Pro Val Trp Ala Ala Ala Ser
               20                  25                  30
   cct ngg gga ngc nca ata ctc gac tcg ggc caa act tgg aac ctt aat   145
   Pro Xaa Gly Xaa Xaa Ile Leu Asp Ser Gly Gln Thr Trp Asn Leu Asn
           35                  40                  45
   gtn aac ccn gga nca atc cag gct cgc att tgg ggc cga acc aat tgt   193
   Val Asn Pro Gly Xaa Ile Gln Ala Arg Ile Trp Gly Arg Thr Asn Cys
       50                  55                  60
   aac ttt gat ggt agt ggc cga ngc nna tac gan acn gga gat tgt tnt   241
   Asn Phe Asp Gly Ser Gly Arg Xaa Xaa Tyr Xaa Xaa Gly Asp Cys Xaa
   65                  70                  75                  80
   ggg ctg tta gaa tgt caa ggt tat ggt agt cca ccc aac act tta gcc   289
   Gly Leu Leu Glu Cys Gln Gly Tyr Gly Ser Pro Pro Asn Thr Leu Ala
                   85                  90                  95
   gaa ttt gct cta aat can ccc aat aac ttg gat ttt gtc gat ntt tct    337
   Glu Phe Ala Leu Asn Thr Pro Asn Asn Leu Asp Phe Val Asp Xaa Ser
                   100                 105                 110
   cta gtc gat nga ttc aac att cct atg gaa ttc cca atc aac ggt ggg    385
   Leu Val Asp Xaa Phe Asn Ile Pro Met Glu Phe Pro Ile Asn Gly Gly
           115                 120                 125
   tgt cgt aat ctc ttn tgc aac gca cat att aac gac caa tgt cct anc    433
   Cys Arg Asn Leu Xaa Cys Asn Ala His Ile Asn Asp Gln Cys Pro Xaa
       130                 135                 140
   caa tta ntg aca ccc ggc ggg tgt tan aac ccc tgc ncg gtt ttc aag    481
   Gln Leu Xaa Thr Pro Gly Gly Cys Xaa Asn Pro Cys Xaa Val Phe Lys
   145                 150                 155                 160
   aca aat gaa ttt tgt nn                                              498
   Thr Asn Glu Phe Cys
                   165
<210> 38
   <211> 165
   <212> PRT
   <213> Solanum tuberosum
<400> 38
   Phe Leu Tyr Phe Gly Gln Tyr Tyr Xaa Tyr Val Xaa His Xaa Xaa Thr
    1               5                  10                  15
   Xaa Asp Ile Thr Asn Arg Cys Thr Tyr Pro Val Trp Ala Ala Ala Ser
               20                  25                  30
   Pro Xaa Gly Xaa Xaa Ile Leu Asp Ser Gly Gln Thr Trp Asn Leu Asn
           35                  40                  45
   Val Asn Pro Gly Xaa Ile Gln Ala Arg Ile Trp Gly Arg Thr Asn Cys
       50                  55                  60
   Asn Phe Asp Gly Ser Gly Arg Xaa Xaa Tyr Xaa Xaa Gly Asp Cys Xaa
   65                  70                  75                  80
   Gly Leu Leu Glu Cys Gln Gly Tyr Gly Ser Pro Pro Asn Thr Leu Ala
                   85                  90                  95
   Glu Phe Ala Leu Asn Thr Pro Asn Asn Leu Asp Phe Val Asp Xaa Ser
               100                 105                 110
   Leu Val Asp Xaa Phe Asn Ile Pro Met Glu Phe Pro Ile Asn Gly Gly
           115                 120                 125
   Cys Arg Asn Leu Xaa Cys Asn Ala His Ile Asn Asp Gln Cys Pro Xaa
       130                 135                 140
   Gln Leu Xaa Thr Pro Gly Gly Cys Xaa Asn Pro Cys Xaa Val Phe Lys
   145                 150                 155                 160
   Thr Asn Glu Phe Cys
                   165

## Patentansprüche

1. Verfahren zur Erkennung und/oder Charakterisierung der direkten anti-pflanzenpathogenen Wirksamkeit von Agenzien, wobei das Verfahren die Schritte in beliebiger Reihenfolge umfaßt, dass pflanzliche Strukturen mit einem Pathogen und mit einem auf anti-pflanzenpathogene Wirksamkeit zu prüfenden Agens behandelt werden, und wobei die Erkennung beziehungsweise die Charakterisierung der Wirksamkeit des Agens anhand der Veränderung der Expressionsstärke der Gene auf der Ebene der RNA erfolgt, indem das Verfahren die Schritte umfasst, dass
a) eine Genbank mit Gensequenzen angelegt wird, deren Expression sich bei Infektion von Pflanzen mit Pathogenen ändert,
b) pflanzliche Strukturen mit dem auf anti-pflanzenpathogene Wirksamkeit zu prüfenden Agens sowie mit dem Pathogen behandelt werden,
c) die RNA des Behandlungsansatzes von Schritt b) isoliert wird,
d) eine Hybridisierungssonde zu der RNA von Schritt c) hergestellt wird,
e) die in Schritt a) erhaltenen Gensequenzen mit der Sonde von Schritt d) und/oder mit der RNA aus Schritt c) hybridisiert werden und
f) eine Veränderung der Expression der Gensequenzen von Schritt a) nachgewiesen wird.

2. Verfahren nach Anspruch 1, wobei es sich bei der RNA im Schritt c) um mRNA handelt.

3. Verfahren nach Anspruch 1, wobei der Nachweis der Veränderung der Expression der Gensequenzen in Schritt f) mittels einer Hybridisierungstechnik erfolgt.

4. Verfahren nach Anspruch 1, umfassend die Schritte, dass nach Schritt c)
d) eine cDNA zu der RNA von Schritt c) hergestellt wird und e) eine Veränderung der Expression der Gensequenzen von Schritt a) nachgewiesen wird.

5. Verfahren nach Anspruch 4, wobei der Nachweis der Veränderung der Expression der Gensequenzen in Schritt e) mittels einer PCR erfolgt.

6. Verfahren nach einem der Ansprüche 1 bis 5, wobei das Anlegen der Genbank in Schritt a) erfolgt, indem
a-1) pflanzliche Strukturen mit dem Pathogen behandelt werden,
a-2) mRNA aus den Strukturen von a-1), aus nicht-behandelten pflanzlichen Strukturen und aus dem Pathogen isoliert wird,
a-3) cDNA der in Schritt a-2) erhaltenen mRNA hergestellt wird,
a-4) aus der cDNA von Schritt a-3) unter Einsatz von subtraktiver Hybridisierung und Suppressions-PCR die Genbank angelegt wird.

7. Verfahren nach einem der vorhergehenden Ansprüche, wobei es sich bei dem Pathogen um Insekten, Viren, Bakterien, Pilze oder Nematoden handelt.

8. Verfahren nach einem der vorhergehenden Ansprüche, wobei das auf seine anti-pflanzenpathogene Wirksamkeit zu prüfende Agens ein Fungizid, Insektizid, Bakterizid, ein virotoxischer Stoff oder ein nematotoxischer Stoff oder eine Zusammensetzung sein kann, die einen oder mehrere dieser Stoffe enthält.

9. Verfahren nach einem der vorhergehenden Ansprüche, wobei es sich bei der geändert exprimierten Gensequenz um ein pflanzliches Gen, insbesondere ein Gen der pflanzlichen Abwehrantwort, ein wundinduziertes Gen und/oder ein stressinduziertes Gen oder deren Gene mit identischer Funktion in verschiedenen Organismen handelt.

10. Verfahren nach einem der vorhergehenden Ansprüche, wobei es sich bei der Gensequenz um ein *in planta* differentiell exprimiertes Gen des Pathogens handelt.

## Claims

1. A method for recognition and/or characterisation of the direct anti-plant pathogen activity of agents, wherein the process comprises the steps in an arbitrary order, wherein plant structures having a pathogen and having an agent to be tested for anti-plant pathogen activity are treated, and wherein the recognition and the characterisation of the activity of said agent, respectively, is performed by means of the modification of expression strength of the genes on the level of the RNA, wherein the method comprises the steps:
a) a gene library having gene sequences is set up, the expression of which is modified on infection of plants by pathogens,
b) plant structures are treated with an agent to be tested for anti-plant pathogen activity as well as with the pathogen,
c) the RNA of the treatment of step b) is isolated,
d) a hybridisation probe to the RNA of step c) is prepared,
e) the gene sequences obtained in step a) by the probe of step d) and/or by the RNA from step c) are hybridised, and
f) a modification of expression of the gene sequences from step a) is detected.

2. The method of claim 1, wherein in step c) the RNA is an mRNA.

3. The method of claim 1, wherein in step f) the detection of the modification of expression of the gene sequences is performed by means of a hybridisation technique.

4. The method of claim 1, comprising the steps, wherein following step c)
d) a cDNA for the RNA of step c) is prepared, and
e) a modification of expression of the gene sequences of step a) is detected.

5. The method of claim 4, wherein in step e) the detection of the modification of expression of the gene sequences is performed by means of PCR.

6. The method according to any one of claims 1 to 5, wherein the setup of the gene library in step a) is performed by
a-1) treating plant structures with the pathogen,
a-2) isolating mRNA from the structures of a-1), from the non-treated plant structures and from the pathogen,
a-3) preparing cDNA of the mRNA obtained in step a-2),
a-4) setup of the gene library from the cDNA of step a-3) by means of subtractive hybridisation and suppression PCR.

7. The method according to any one of the preceding claims, wherein the pathogen is an insect, a virus, a bacterium, a fungus or a nematode.

8. The method according to any one of the preceding claims, wherein the agent to be tested for anti-plant pathogen activity may be a fungicide, an insecticide, a bactericide, a virotoxic substance or a nematotoxic substance or a composition containing one or more of these substances.

9. The method according to any one of the preceding claims, wherein the modified expressed gene sequence is a plant gene, in particular a gene of the plant resistance response, a wound-induced gene and/or a stress-induced gene or the genes thereof having an identical function in various organisms.

10. The method according to any one of the preceding claims, wherein the gene sequence is an *in planta* differentially expressed gene of the pathogen.

## Revendications

1. Procédé pour reconnaître et/ou caractériser l'efficacité directe d'agents contre des pathogènes de plantes, ledit procédé comprenant - dans un ordre quelconque - les étapes consistant en ce que des structures végétales sont traitées avec un pathogène et avec un agent à examiner quant à l'efficacité contre des pathogènes de plantes, et la reconnaissance ou bien la caractérisation de l'efficacité dudit agent se faisant au moyen de la modification du pouvoir d'expression des gènes au niveau de l'ARN, le procédé comprenant les étapes consistant en ce que
a) on établit une banque de gènes avec des séquences de gènes dont l 'expression change lors d'une infection de plantes par des pathogènes,
b) on traite des structures végétales avec ledit agent à examiner quant à l 'efficacité contre des pathogènes de plantes ainsi qu'avec le pathogène,
c) on isole l'ARN du traitement de l'étape b),
d) on réalise une sonde d'hybridation relative à l'ARN de l'étape c),
e) les séquences de gènes obtenues dans l'étape a) sont hybridées avec la sonde de l'étape d) et/ou avec l'ARN de l'étape c), et
f) on détecte une modification de l'expression des séquences de gènes de l 'étape a).

2. Procédé selon la revendication 1, l'ARN dans l'étape c) étant de l'ARNm.

3. Procédé selon la revendication 1, la détection de la modification de l'expression des séquences de gènes dans l'étape f) se faisant au moyen d'une technique d 'hybridation.

4. Procédé selon la revendication 1, comprenant les étapes consistant en ce que, après l'étape c)
d) on réalise un ADNc rélatif à l'ARN de l'étape c), et
e) on détecte une modification de l'expression des séquences de gènes de I 'étape a).

5. Procédé selon la revendication 4, la détection de la modification de l'expression des séquences de gènes dans l'étape e) se faisant au moyen d'une PCR.

6. Procédé selon l'une des revendications 1 à 5, l'établissement de la banque de gènes dans l'étape a) se faisant
a-1) en traitant des structures végétales avec le pathogène,
a-2) en isolant de l'ARNm des structures de a-1), de structures végétales non traitées et du pathogène,
a-3) en réalisant de l'ADNc de l'ARNm obtenu dans l'étape a-2),
a-4) en établissant la banque de gènes à partir de l'ADNc de l'étape a-3) en mettant en oeuvre l'hybridation soustractive et la PCR suppressive.

7. Procédé selon l'une des revendications précédentes, le pathogène étant constitué par des insectes, des virus, des bactéries, des champignons ou des nématodes.

8. Procédé selon l'une des revendications précédentes, l'agent à examiner quant à son efficacité contre des pathogènes de plantes pouvant être un fongicide, un insecticide, un bactéricide, une substance viro-toxique ou une substance némato-toxique ou une composition qui contient une ou plusieurs de ces substances.

9. Procédé selon l'une des revendications précédentes, la séquence de gène exprimée de façon modifiée étant un gène végétal, en particulier un gène de la réponse de défense végétale, un gène induit par blessure et/ou un gène induit par le stress ou leurs gènes à fonction identique dans des organismes différents.

10. Procédé selon l'une des revendications précédentes, la séquence de gène étant un gène du pathogène, qui est différentiellement exprimé *in planta.*
